# EUROPEAN PATENT APPLICATION

(11) **EP 4 847 614 A1**
(43) Date of publication of application: **02.12.2026**
(21) Application number: 25744734.2
(22) Date of filing: 24.01.2025
(51) Int. Cl.: C12N 15/113, A61P 7/02, A61K 31/713, A61K 48/00

(54) **NUCLEIC ACID MOLECULE INHIBITING F11 GENE EXPRESSION**

(30) Priority: 26.01.2024 CN 202410114595; 09.05.2024 CN 202410568807
(71) Applicant: CSPC Zhongqi Pharmaceutical Technology (Shijiazhuang) Co., Ltd., Shijiazhuang, Hebei 050035 (CN)
(72) Inventor: ZHANG, Xueyan, Shijiazhuang, Hebei 050035 (CN); SHI, Yanjun, Shijiazhuang, Hebei 050035 (CN); WANG, Lingyu, Shijiazhuang, Hebei 050035 (CN); DONG, Yunxia, Shijiazhuang, Hebei 050035 (CN); ZHAO, Chenglong, Shijiazhuang, Hebei 050035 (CN); ZHONG, Qiang, Shijiazhuang, Hebei 050035 (CN); SHAO, Yu, Shijiazhuang, Hebei 050035 (CN); LIU, Jianguo, Shijiazhuang, Hebei 050035 (CN); WANG, Chuanjin, Shijiazhuang, Hebei 050035 (CN); XU, Hailiang, Shijiazhuang, Hebei 050035 (CN); SU, Xiaoye, Shijiazhuang, Hebei 050035 (CN); LI, Chunlei, Shijiazhuang, Hebei 050035 (CN)
(74) Representative: Gille Hrabal Partnerschaftsgesellschaft mbB Patentanwälte
(86) International application number: PCT/CN2025/074764
(87) International publication number: WO 2025/157271

(57) **Abstract**

The present invention relates to a nucleic acid molecule inhibiting factor 11 (FXI) gene expression in a cell by means of RNAi, comprising a sense sequence and an antisense sequence complementary to each other, or consisting of a sense sequence and an antisense sequence complementary to each other. The FXI inhibition rate of the nucleic acid molecule is significantly superior to that of other small nucleic acid molecules which inhibit the expression of FXI by means of RNAi.

## Description

The present application claims priority to the following prior applications: Patent Application No. 202410114595.7, filed with the China National Intellectual Property Administration on January 26, 2024, entitled "Nucleic Acid Molecule Inhibiting F11 Gene Expression"; and Patent Application No. 202410568807.9, filed with the China National Intellectual Property Administration on May 9, 2024, entitled "GalNAc Derivative and Oligonucleotide Conjugate Thereof". The two prior applications are incorporated herein by reference in their entirety.

### TECHNICAL FIELD

The present application relates to the field of RNAi, specifically to a nucleic acid molecule that can inhibit the expression of the F11 gene by means of RNAi and uses thereof.

### BACKGROUND

RNA interference (RNAi) is a highly conserved evolutionary phenomenon characterized by the efficient and specific degradation of homologous mRNA induced by doublestranded RNA (dsRNA). dsRNA, typically 19-30 bp in length, is one of the important tools in RNAi technology. In natural organisms, long dsRNAs are usually recognized by the Dicer enzyme after entering the cell and cleaved into small RNA fragments (i.e., siRNAs) about 21 to 23 nucleotides long. The resulting dsRNA fragments unwind into single strands and form complexes with certain proteins (RISCs for short). RISC can bind to mRNA that is complementary to the antisense strand of dsRNA in the cell and cleave the mRNA, causing it to be degraded, resulting in the inability to synthesize proteins and producing a gene "silencing" phenomenon. In industrial production, people tend to chemically synthesize dsRNA and modify it to further improve the stability and efficacy of dsRNA drugs.

A thrombus is a blood clot that restricts blood flow within a blood vessel. It can occur in the arterial or venous circulation and is the common pathological basis for most myocardial infarctions, ischemic strokes, and venous thromboembolism (VTE). Currently, the main targets of commonly used anticoagulants are thrombin and FX (or coagulation factor 10), which are equally important in hemostasis and thrombosis. If an attempt is made to exert an anticoagulant effect by inhibiting these two factors, it will inevitably affect the hemostasis process. This contradiction limits the intensity of anticoagulant therapy, meaning that patients at high risk of bleeding may not be suitable for anticoagulation treatment. Clinically, there is a need for new anticoagulants that can reduce the risk of bleeding while achieving anticoagulation, and coagulation factor XI (FXI) has become a new anticoagulation target. Compared to FX, FXI (or coagulation factor FXI or F11) plays an auxiliary role in hemostasis but a necessary role in thrombosis. Spontaneous hemorrhage, central nervous system hemorrhage or gastrointestinal hemorrhage is rarely observed even in patients with severe FXI deficiency.

### SUMMARY

The present application provides a nucleic acid molecule that inhibits the expression of cytokine XI (FXI) gene via RNAi, as well as compositions, formulations, and uses of the nucleic acid molecule. Sufficient data in the examples of the present application demonstrate that preferred nucleic acid molecules of the present application include, but are not limited to, dsRNA molecules numbered 3, 3', 5, 8, 9, 9', 14, 14', 15, 19, 24, 24', 26, 26', 50, 70, 70' and 82; shRNA molecules whose structure and sequence comprise the aforementioned dsRNA molecules; precursor molecules of the shRNA molecules or dsRNA molecules; and modified forms of the shRNA molecules or dsRNA molecules. Preferred modifications include, but are not limited to, modification E and modification E-1 described in detail in the examples herein.

Specifically, in one aspect, the present application relates to a nucleic acid molecule which comprises or consists of a sense sequence and an antisense sequence complementary to each other, and the antisense sequence comprises a polynucleotide sequence complementary to FXI gene mRNA, wherein
when the antisense sequence hybridizes with the FXI gene mRNA at the maximum complementarity, the base pairs formed by complementary pairing between the antisense sequence and the FXI gene mRNA sequence constitute complementary region 1;
when the antisense sequence hybridizes with the sense sequence at the maximum complementarity, the base pairs formed by complementary pairing between the antisense sequence and the sense sequence constitute complementary region 2;
the complementary region 1 and the complementary region 2 share at least 18, 19 or 20 identical base pairs,
the number of base pairs in the complementary region 1 is 15 to 35 bp, and the complementary region 1 comprises the following nucleotides in the FXI gene mRNA sequence:
   nucleotides at positions 418 to 437, or 18 or 19 consecutive nucleotides therein,
   nucleotides at positions 1141 to 1160, or 18 or 19 consecutive nucleotides therein,
   nucleotides at positions 363 to 382, or 18 or 19 consecutive nucleotides therein,
   nucleotides at positions 372 to 391, or 18 or 19 consecutive nucleotides therein,
   nucleotides at positions 411 to 430, or 18 or 19 consecutive nucleotides therein,
   nucleotides at positions 421 to 440, or 18 or 19 consecutive nucleotides therein,
   nucleotides at positions 422 to 441, or 18 or 19 consecutive nucleotides therein,
   nucleotides at positions 425 to 444, or 18 or 19 consecutive nucleotides therein,
   nucleotides at positions 465 to 484, or 18 or 19 consecutive nucleotides therein,
   nucleotides at positions 470 to 489, or 18 or 19 consecutive nucleotides therein,
   nucleotides at positions 471 to 490, or 18 or 19 consecutive nucleotides therein, or
   nucleotides at positions 518 to 537, or 18 or 19 consecutive nucleotides therein; and
   the nucleotide positions of the FXI gene mRNA sequence are numbered in accordance with the corresponding nucleotides in the reference sequence of SEQ ID NO: 244.

"Consecutive 18 or 19 nucleotides therein" within a given nucleotide sequence refers to consecutive 19 nucleotides starting from the first or second nucleotide at the 5' end of the nucleotide sequence, including the first nucleotide or second nucleotide and retaining the original nucleotide order of the nucleotide sequence; or consecutive 18 nucleotides starting from the first, second or third nucleotide at the 5' end of the nucleotide sequence, including the first, second or third nucleotide and retaining the original nucleotide order of the nucleotide sequence.

In some embodiments, the sense sequence and the antisense sequence have the same length. In some embodiments, the sense sequence and/or the antisense sequence further comprise overhang nucleotides outside the complementary region 2. In some embodiments, the sense sequence is longer than the antisense sequence. In some embodiments, the antisense sequence is longer than the sense sequence. In some embodiments, the sense sequence is longer than the antisense sequence by 1, 2, 3 or 4 nucleotides. In some embodiments, the antisense sequence is longer than the sense sequence by 1, 2, 3 or 4 nucleotides. In some embodiments, the sense sequence further comprises 1 or 2 nucleotides on the 5' side and/or the 3' side of the complementary region 2. In some embodiments, the antisense sequence further comprises 1 or 2 nucleotides on the 5' side and/or the 3' side of the complementary region 2. In some embodiments, both the sense sequence and the antisense sequence further comprise 1 or 2 nucleotides on the 5' side and/or the 3' side of the complementary region 2. In some embodiments, when the antisense sequence hybridizes with the sense sequence at maximum complementarity, the sense sequence does not comprise any nucleotides that are not complementary to the antisense sequence nucleotides between the first nucleotide and the last nucleotide of the complementary region 2. In some embodiments, when the antisense sequence hybridizes with the sense sequence at maximum complementarity, the antisense sequence does not comprise any nucleotides that are not complementary to the sense sequence nucleotides between the first nucleotide and the last nucleotide of the complementary region 2. In some embodiments, there are 2 overhang nucleotides located in the antisense sequence and adjacent to the 5' end of the complementary region 2, and the sense sequence comprises no overhang nucleotides. In some embodiments, the antisense sequence comprises only 0, 1 or 2 nucleotides outside the complementary region 1, and the sense sequence comprises only 0, 1 or 2 nucleotides outside the complementary region 2. In some embodiments, when the antisense sequence hybridizes with the mRNA sequence of the FXI gene at the maximum complementarity, the antisense sequence does not comprise nucleotides that fail to form complementary pairing with the mRNA sequence of the FXI gene between the first nucleotide and the last nucleotide of the complementary region 1. In some embodiments, when the antisense sequence hybridizes with the mRNA sequence of the FXI gene at the maximum complementarity, the mRNA sequence of the FXI gene does not comprise nucleotides that fail to form complementary pairing with the antisense sequence between the first nucleotide and the last nucleotide of the complementary region 1. In some embodiments, the 3' end of the complementary region 2 is an AU base pair. In some embodiments, the 3' end of the complementary region 2 is composed of A derived from the antisense sequence and U derived from the sense sequence (U may be written as T in the present application). In some embodiments, the 3' end of the complementary region 2 is composed of A derived from the sense sequence and U derived from the antisense sequence (U may be written as T in the present application). In some embodiments, the complementary region 1 and the complementary region 2 are uninterrupted, that is, continuous. In some embodiments, the complementary region 1 and/or complementary region 2 are discontinuous, and such discontinuity means being interrupted by one or more bubbles (a bubble refers to a non-complementary region formed by respective non-complementary nucleotides in the two sequences that constitute the complementary region).

In some embodiments, the complementary region 1 has 15 to 35 base pairs, for example 15, 16, 17, 18, 19, 20, 21, 22, 23, 24, 25, 26, 27, 28, 29, 30, 31, 32, 33, 34 or 35 bp. In some embodiments, the complementary region 2 has 15 to 35 base pairs (bp), for example 15, 16, 17, 18, 19, 20, 21, 22, 23, 24, 25, 26, 27, 28, 29, 30, 31, 32, 33, 34 or 35 bp. In some embodiments, the complementary region 1 and complementary region 2 have 18, 19, 20, 21, 22 or 23 identical base pairs. In some embodiments, the length of the sense sequence and/or the antisense sequence is 15, 16, 17, 18, 19, 20, 21, 22, 23, 24, 25, 26, 27, 28, 29 or 30 nt. In some embodiments, the length of the sense sequence is 21 nt, and the length of the antisense sequence is 23 nt. In some embodiments, the nucleic acid molecule consists of a sense sequence of 21 nt in length and an antisense sequence of 23 nt in length. In some embodiments, the nucleic acid molecule consists of a sense sequence, an antisense sequence and a linker strand (also referred to as shRNA loop), wherein the linker strand connects the 3'-end nucleotide of the sense sequence and the 5'-end nucleotide of the antisense sequence. The selection of the linker strand is conventional in the art, for example, it may be selected in accordance with the method described in Jensen, Stig Mølgaard Rask et al. "Functional selection of shRNA loops from randomized retroviral libraries." PLoS one vol. 7,8 (2012): e43095. doi:10.1371/journal.pone.0043095, which is herein incorporated by reference in its entirety.

In some embodiments, the sense sequence and/or the antisense sequence further comprise 1 to 2 overhang nucleotides outside the complementary region 2.

In some embodiments, the antisense sequence comprises only 0, 1 or 2 nucleotides outside the complementary region 1, and the sense sequence comprises only 0, 1 or 2 nucleotides outside the complementary region 2.

In some embodiments, the 3' end of the complementary region 2 is an AU base pair. In further embodiments, the 5'-end A of the antisense strand is located outside the complementary region 1.

In some embodiments, the FXI gene mRNA sequence complementary to the antisense strand is transcribed from the coding region of a mammalian FXI gene. In some embodiments, the mammals is a primate. In some embodiments, the primate is a human or a cynomolgus monkey. In some embodiments, the mRNA comprises the polynucleotide sequence set forth in SEQ ID NO: 244. In some embodiments, the antisense sequence comprises the polynucleotide sequence set forth in SEQ ID NO: 122, 123, 126, 129, 130, 131, 136, 137, 138, 143, 149, 150, 152, 153, 177, 200, 201 or 213. In some embodiments, the antisense sequence consists of the polynucleotide sequence set forth in SEQ ID NO: 122, 123, 126, 129, 130, 131, 136, 137, 138, 143, 149, 150, 152, 153, 177, 200, 201 or 213 plus additional 1, 2, 3 or 4 nucleotides. In some embodiments, the antisense sequence consists of the polynucleotide sequence set forth in SEQ ID NO: 122, 123, 126, 129, 130, 131, 136, 137, 138, 143, 149, 150, 152, 153, 177, 200, 201 or 213 plus additional 1 to 2 nucleotides on the 5' and/or 3' side of the polynucleotide sequence. In some embodiments, the antisense sequence is the polynucleotide sequence set forth in SEQ ID NO: 122, 123, 126, 129, 130, 131, 136, 137, 138, 143, 149, 150, 152, 153, 177, 200, 201 or 213. In some embodiments, the sense sequence comprises the polynucleotide sequence set forth in SEQ ID NO: 3, 4, 7, 10, 11, 12, 17, 18, 19, 24, 30, 31, 33, 34, 58, 81, 82 or 94. In some embodiments, the antisense sequence consists of the polynucleotide sequence set forth in SEQ ID NO: 3, 4, 7, 10, 11, 12, 17, 18, 19, 24, 30, 31, 33, 34, 58, 81, 82 or 94 plus additional 1 to 4 (e.g., 2 or 3) nucleotides. In some embodiments, the antisense sequence consists of the polynucleotide sequence set forth in SEQ ID NO: 3, 4, 7, 10, 11, 12, 17, 18, 19, 24, 30, 31, 33, 34, 58, 81, 82 or 94 plus additional 1 to 2 nucleotides on the 5' and/or 3' side of the polynucleotide sequence. In some embodiments, the antisense sequence is the polynucleotide sequence set forth in SEQ ID NO: 3, 4, 7, 10, 11, 12, 17, 18, 19, 24, 30, 31, 33, 34, 58, 81, 82 or 94.

In some embodiments, the sense sequence comprises or is the polynucleotide sequence set forth in SEQ ID NO: 3, and the antisense sequence comprises or is the polynucleotide sequence set forth in SEQ ID NO: 122; the sense sequence comprises or is the polynucleotide sequence set forth in SEQ ID NO: 4, and the antisense sequence comprises or is the polynucleotide sequence set forth in SEQ ID NO: 123; the sense sequence comprises or is the polynucleotide sequence set forth in SEQ ID NO: 7, and the antisense sequence comprises or is the polynucleotide sequence set forth in SEQ ID NO: 126; the sense sequence comprises or is the polynucleotide sequence set forth in SEQ ID NO: 10, and the antisense sequence comprises or is the polynucleotide sequence set forth in SEQ ID NO: 129; the sense sequence comprises or is the polynucleotide sequence set forth in SEQ ID NO: 11, and the antisense sequence comprises or is the polynucleotide sequence set forth in SEQ ID NO: 130; the sense sequence comprises or is the polynucleotide sequence set forth in SEQ ID NO: 12, and the antisense sequence comprises or is the polynucleotide sequence set forth in SEQ ID NO: 131; the sense sequence comprises or is the polynucleotide sequence set forth in SEQ ID NO: 17, and the antisense sequence comprises or is the polynucleotide sequence set forth in SEQ ID NO: 136; the sense sequence comprises or is the polynucleotide sequence set forth in SEQ ID NO: 18, and the antisense sequence comprises or is the polynucleotide sequence set forth in SEQ ID NO: 137; the sense sequence comprises or is the polynucleotide sequence set forth in SEQ ID NO: 19, and the antisense sequence comprises or is the polynucleotide sequence set forth in SEQ ID NO: 138; the sense sequence comprises or is the polynucleotide sequence set forth in SEQ ID NO: 24, and the antisense sequence comprises or is the polynucleotide sequence set forth in SEQ ID NO: 143; the sense sequence comprises or is the polynucleotide sequence set forth in SEQ ID NO: 30, and the antisense sequence comprises or is the polynucleotide sequence set forth in SEQ ID NO: 149; the sense sequence comprises or is the polynucleotide sequence set forth in SEQ ID NO: 31, and the antisense sequence comprises or is the polynucleotide sequence set forth in SEQ ID NO: 150; the sense sequence comprises or is the polynucleotide sequence set forth in SEQ ID NO: 33, and the antisense sequence comprises or is the polynucleotide sequence set forth in SEQ ID NO: 152; the sense sequence comprises or is the polynucleotide sequence set forth in SEQ ID NO: 34, and the antisense sequence comprises or is the polynucleotide sequence set forth in SEQ ID NO: 153; the sense sequence comprises or is the polynucleotide sequence set forth in SEQ ID NO: 58, and the antisense sequence comprises or is the polynucleotide sequence set forth in SEQ ID NO: 177; the sense sequence comprises or is the polynucleotide sequence set forth in SEQ ID NO: 81, and the antisense sequence comprises or is the polynucleotide sequence set forth in SEQ ID NO: 200; the sense sequence comprises or is the polynucleotide sequence set forth in SEQ ID NO: 82, and the antisense sequence comprises or is the polynucleotide sequence set forth in SEQ ID NO: 201; or the sense sequence comprises or is the polynucleotide sequence set forth in SEQ ID NO: 94, and the antisense sequence comprises or is the polynucleotide sequence set forth in SEQ ID NO: 213.

In some embodiments, the sense sequence consists of the polynucleotide sequence set forth in SEQ ID NO: 3 plus additional 1, 2, 3 or 4 nucleotides, and the antisense sequence consists of the polynucleotide sequence set forth in SEQ ID NO: 122 plus additional 1 to 4 (e.g., 2 or 3) nucleotides; the sense sequence consists of the polynucleotide sequence set forth in SEQ ID NO: 4 plus additional 1 to 4 (e.g., 2 or 3) nucleotides, and the antisense sequence consists of the polynucleotide sequence set forth in SEQ ID NO: 123 plus additional 1 to 4 (e.g., 2 or 3) nucleotides; the sense sequence consists of the polynucleotide sequence set forth in SEQ ID NO: 7 plus additional 1 to 4 (e.g., 2 or 3) nucleotides, and the antisense sequence consists of the polynucleotide sequence set forth in SEQ ID NO: 126 plus additional 1 to 4 (e.g., 2 or 3) nucleotides; the sense sequence consists of the polynucleotide sequence set forth in SEQ ID NO: 10 plus additional 1 to 4 (e.g., 2 or 3) nucleotides, and the antisense sequence consists of the polynucleotide sequence set forth in SEQ ID NO: 129 plus additional 1 to 4 (e.g., 2 or 3) nucleotides; the sense sequence consists of the polynucleotide sequence set forth in SEQ ID NO: 11 plus additional 1 to 4 (e.g., 2 or 3) nucleotides, and the antisense sequence consists of the polynucleotide sequence set forth in SEQ ID NO: 130 plus additional 1 to 4 (e.g., 2 or 3) nucleotides; the sense sequence consists of the polynucleotide sequence set forth in SEQ ID NO: 12 plus additional 1 to 4 (e.g., 2 or 3) nucleotides, and the antisense sequence consists of the polynucleotide sequence set forth in SEQ ID NO: 131 plus additional 1 to 4 (e.g., 2 or 3) nucleotides; the sense sequence consists of the polynucleotide sequence set forth in SEQ ID NO: 17 plus additional 1 to 4 (e.g., 2 or 3) nucleotides, and the antisense sequence consists of the polynucleotide sequence set forth in SEQ ID NO: 136 plus additional 1 to 4 (e.g., 2 or 3) nucleotides; the sense sequence consists of the polynucleotide sequence set forth in SEQ ID NO: 18 plus additional 1 to 4 (e.g., 2 or 3) nucleotides, and the antisense sequence consists of the polynucleotide sequence set forth in SEQ ID NO: 137 plus additional 1 to 4 (e.g., 2 or 3) nucleotides; the sense sequence consists of the polynucleotide sequence set forth in SEQ ID NO: 19 plus additional 1 to 4 (e.g., 2 or 3) nucleotides, and the antisense sequence consists of the polynucleotide sequence set forth in SEQ ID NO: 138 plus additional 1 to 4 (e.g., 2 or 3) nucleotides; the sense sequence consists of the polynucleotide sequence set forth in SEQ ID NO: 24 plus additional 1 to 4 (e.g., 2 or 3) nucleotides, and the antisense sequence consists of the polynucleotide sequence set forth in SEQ ID NO: 143 plus additional 1 to 4 (e.g., 2 or 3) nucleotides; the sense sequence consists of the polynucleotide sequence set forth in SEQ ID NO: 30 plus additional 1 to 4 (e.g., 2 or 3) nucleotides, and the antisense sequence consists of the polynucleotide sequence set forth in SEQ ID NO: 149 plus additional 1 to 4 (e.g., 2 or 3) nucleotides; the sense sequence consists of the polynucleotide sequence set forth in SEQ ID NO: 31 plus additional 1 to 4 (e.g., 2 or 3) nucleotides, and the antisense sequence consists of the polynucleotide sequence set forth in SEQ ID NO: 150 plus additional 1 to 4 (e.g., 2 or 3) nucleotides; the sense sequence consists of the polynucleotide sequence set forth in SEQ ID NO: 33 plus additional 1 to 4 (e.g., 2 or 3) nucleotides, and the antisense sequence consists of the polynucleotide sequence set forth in SEQ ID NO: 152 plus additional 1 to 4 (e.g., 2 or 3) nucleotides; the sense sequence consists of the polynucleotide sequence set forth in SEQ ID NO: 34 plus additional 1 to 4 (e.g., 2 or 3) nucleotides, and the antisense sequence consists of the polynucleotide sequence set forth in SEQ ID NO: 153 plus additional 1 to 4 (e.g., 2 or 3) nucleotides; the sense sequence consists of the polynucleotide sequence set forth in SEQ ID NO: 58 plus additional 1 to 4 (e.g., 2 or 3) nucleotides, and the antisense sequence consists of the polynucleotide sequence set forth in SEQ ID NO: 177 plus additional 1 to 4 (e.g., 2 or 3) nucleotides; the sense sequence consists of the polynucleotide sequence set forth in SEQ ID NO: 81 plus additional 1 to 4 (e.g., 2 or 3) nucleotides, and the antisense sequence consists of the polynucleotide sequence set forth in SEQ ID NO: 200 plus additional 1 to 4 (e.g., 2 or 3) nucleotides; the sense sequence consists of the polynucleotide sequence set forth in SEQ ID NO: 82 plus additional 1 to 4 (e.g., 2 or 3) nucleotides, and the antisense sequence consists of the polynucleotide sequence set forth in SEQ ID NO: 201 plus additional 1 to 4 (e.g., 2 or 3) nucleotides; or the sense sequence consists of the polynucleotide sequence set forth in SEQ ID NO: 94 plus additional 1 to 4 (e.g., 2 or 3) nucleotides, and the antisense sequence consists of the polynucleotide sequence set forth in SEQ ID NO: 213 plus additional 1 to 4 (e.g., 2 or 3) nucleotides.

In some embodiments, the sense sequence consists of the polynucleotide sequence set forth in SEQ ID NO: 3 plus additional 1 to 4 (e.g., 2 or 3) nucleotides, and the antisense sequence is the polynucleotide sequence set forth in SEQ ID NO: 122; the sense sequence consists of the polynucleotide sequence set forth in SEQ ID NO: 4 plus additional 1 to 4 (e.g., 2 or 3) nucleotides, and the antisense sequence is the polynucleotide sequence set forth in SEQ ID NO: 123; the sense sequence consists of the polynucleotide sequence set forth in SEQ ID NO: 7 plus additional 1 to 4 (e.g., 2 or 3) nucleotides, and the antisense sequence is the polynucleotide sequence set forth in SEQ ID NO: 126; the sense sequence consists of the polynucleotide sequence set forth in SEQ ID NO: 10 plus additional 1 to 4 (e.g., 2 or 3) nucleotides, and the antisense sequence is the polynucleotide sequence set forth in SEQ ID NO: 129; the sense sequence consists of the polynucleotide sequence set forth in SEQ ID NO: 11 plus additional 1 to 4 (e.g., 2 or 3) nucleotides, and the antisense sequence is the polynucleotide sequence set forth in SEQ ID NO: 130; the sense sequence consists of the polynucleotide sequence set forth in SEQ ID NO: 12 plus additional 1 to 4 (e.g., 2 or 3) nucleotides, and the antisense sequence is the polynucleotide sequence set forth in SEQ ID NO: 131; the sense sequence consists of the polynucleotide sequence set forth in SEQ ID NO: 17 plus additional 1 to 4 (e.g., 2 or 3) nucleotides, and the antisense sequence is the polynucleotide sequence set forth in SEQ ID NO: 136; the sense sequence consists of the polynucleotide sequence set forth in SEQ ID NO: 18 plus additional 1 to 4 (e.g., 2 or 3) nucleotides, and the antisense sequence is the polynucleotide sequence set forth in SEQ ID NO: 137; the sense sequence consists of the polynucleotide sequence set forth in SEQ ID NO: 19 plus additional 1 to 4 (e.g., 2 or 3) nucleotides, and the antisense sequence is the polynucleotide sequence set forth in SEQ ID NO: 138; the sense sequence consists of the polynucleotide sequence set forth in SEQ ID NO: 24 plus additional 1 to 4 (e.g., 2 or 3) nucleotides, and the antisense sequence is the polynucleotide sequence set forth in SEQ ID NO: 143; the sense sequence consists of the polynucleotide sequence set forth in SEQ ID NO: 30 plus additional 1 to 4 (e.g., 2 or 3) nucleotides, and the antisense sequence is the polynucleotide sequence set forth in SEQ ID NO: 149; the sense sequence consists of the polynucleotide sequence set forth in SEQ ID NO: 31 plus additional 1 to 4 (e.g., 2 or 3) nucleotides, and the antisense sequence is the polynucleotide sequence set forth in SEQ ID NO: 150; the sense sequence consists of the polynucleotide sequence set forth in SEQ ID NO: 33 plus additional 1 to 4 (e.g., 2 or 3) nucleotides, and the antisense sequence is the polynucleotide sequence set forth in SEQ ID NO: 152; the sense sequence consists of the polynucleotide sequence set forth in SEQ ID NO: 34 plus additional 1 to 4 (e.g., 2 or 3) nucleotides, and the antisense sequence is the polynucleotide sequence set forth in SEQ ID NO: 153; the sense sequence consists of the polynucleotide sequence set forth in SEQ ID NO: 58 plus additional 1 to 4 (e.g., 2 or 3) nucleotides, and the antisense sequence is the polynucleotide sequence set forth in SEQ ID NO: 177; the sense sequence consists of the polynucleotide sequence set forth in SEQ ID NO: 81 plus additional 1 to 4 (e.g., 2 or 3) nucleotides, and the antisense sequence is the polynucleotide sequence set forth in SEQ ID NO: 200; the sense sequence consists of the polynucleotide sequence set forth in SEQ ID NO: 82 plus additional 1 to 4 (e.g., 2 or 3) nucleotides, and the antisense sequence is the polynucleotide sequence set forth in SEQ ID NO: 201; or the sense sequence consists of the polynucleotide sequence set forth in SEQ ID NO: 94 plus additional 1 to 4 (e.g., 2 or 3) nucleotides, and the antisense sequence is the polynucleotide sequence set forth in SEQ ID NO: 213.

In some embodiments, the sense sequence is the polynucleotide sequence set forth in SEQ ID NO: 3, and the antisense sequence consists of the polynucleotide sequence set forth in SEQ ID NO: 122 plus additional 1 to 4 (e.g., 2 or 3) nucleotides; the sense sequence is the polynucleotide sequence set forth in SEQ ID NO: 4, and the antisense sequence consists of the polynucleotide sequence set forth in SEQ ID NO: 123 plus additional 1 to 4 (e.g., 2 or 3) nucleotides; the sense sequence is the polynucleotide sequence set forth in SEQ ID NO: 7, and the antisense sequence consists of the polynucleotide sequence set forth in SEQ ID NO: 126 plus additional 1 to 4 (e.g., 2 or 3) nucleotides; the sense sequence is the polynucleotide sequence set forth in SEQ ID NO: 10, and the antisense sequence consists of the polynucleotide sequence set forth in SEQ ID NO: 129 plus additional 1 to 4 (e.g., 2 or 3) nucleotides; the sense sequence is the polynucleotide sequence set forth in SEQ ID NO: 11, and the antisense sequence consists of the polynucleotide sequence set forth in SEQ ID NO: 130 plus additional 1 to 4 (e.g., 2 or 3) nucleotides; the sense sequence is the polynucleotide sequence set forth in SEQ ID NO: 12, and the antisense sequence consists of the polynucleotide sequence set forth in SEQ ID NO: 131 plus additional 1 to 4 (e.g., 2 or 3) nucleotides; the sense sequence is the polynucleotide sequence set forth in SEQ ID NO: 17, and the antisense sequence consists of the polynucleotide sequence set forth in SEQ ID NO: 136 plus additional 1 to 4 (e.g., 2 or 3) nucleotides; the sense sequence is the polynucleotide sequence set forth in SEQ ID NO: 18, and the antisense sequence consists of the polynucleotide sequence set forth in SEQ ID NO: 137 plus additional 1 to 4 (e.g., 2 or 3) nucleotides; the sense sequence is the polynucleotide sequence set forth in SEQ ID NO: 19, and the antisense sequence consists of the polynucleotide sequence set forth in SEQ ID NO: 138 plus additional 1 to 4 (e.g., 2 or 3) nucleotides; the sense sequence is the polynucleotide sequence set forth in SEQ ID NO: 24, and the antisense sequence consists of the polynucleotide sequence set forth in SEQ ID NO: 143 plus additional 1 to 4 (e.g., 2 or 3) nucleotides; the sense sequence is the polynucleotide sequence set forth in SEQ ID NO: 30, and the antisense sequence consists of the polynucleotide sequence set forth in SEQ ID NO: 149 plus additional 1 to 4 (e.g., 2 or 3) nucleotides; the sense sequence is the polynucleotide sequence set forth in SEQ ID NO: 31, and the antisense sequence consists of the polynucleotide sequence set forth in SEQ ID NO: 150 plus additional 1 to 4 (e.g., 2 or 3) nucleotides; the sense sequence is the polynucleotide sequence set forth in SEQ ID NO: 33, and the antisense sequence consists of the polynucleotide sequence set forth in SEQ ID NO: 152 plus additional 1 to 4 (e.g., 2 or 3) nucleotides; the sense sequence is the polynucleotide sequence set forth in SEQ ID NO: 34, and the antisense sequence consists of the polynucleotide sequence set forth in SEQ ID NO: 153 plus additional 1 to 4 (e.g., 2 or 3) nucleotides; the sense sequence is the polynucleotide sequence set forth in SEQ ID NO: 58, and the antisense sequence consists of the polynucleotide sequence set forth in SEQ ID NO: 177 plus additional 1 to 4 (e.g., 2 or 3) nucleotides; the sense sequence is the polynucleotide sequence set forth in SEQ ID NO: 81, and the antisense sequence consists of the polynucleotide sequence set forth in SEQ ID NO: 200 plus additional 1 to 4 (e.g., 2 or 3) nucleotides; the sense sequence is the polynucleotide sequence set forth in SEQ ID NO: 82, and the antisense sequence consists of the polynucleotide sequence set forth in SEQ ID NO: 201 plus additional 1 to 4 (e.g., 2 or 3) nucleotides; or the sense sequence is the polynucleotide sequence set forth in SEQ ID NO: 94, and the antisense sequence consists of the polynucleotide sequence set forth in SEQ ID NO: 213 plus additional 1 to 4 (e.g., 2 or 3) nucleotides.

In some embodiments:
the sense sequence comprises or is the polynucleotide sequence set forth in SEQ ID NO: 34, and the antisense sequence comprises or is the polynucleotide sequence set forth in SEQ ID NO: 153;
the sense sequence comprises or is the polynucleotide sequence set forth in SEQ ID NO: 58, and the antisense sequence comprises or is the polynucleotide sequence set forth in SEQ ID NO: 177; the sense sequence comprises or is the polynucleotide sequence set forth in SEQ ID NO: 33, and the antisense sequence comprises or is the polynucleotide sequence set forth in SEQ ID NO: 152;
the sense sequence comprises or is the polynucleotide sequence set forth in SEQ ID NO: 3, and the antisense sequence comprises or is the polynucleotide sequence set forth in SEQ ID NO: 122;
the sense sequence comprises or is the polynucleotide sequence set forth in SEQ ID NO: 4, and the antisense sequence comprises or is the polynucleotide sequence set forth in SEQ ID NO: 123;
the sense sequence comprises or is the polynucleotide sequence set forth in SEQ ID NO: 7, and the antisense sequence comprises or is the polynucleotide sequence set forth in SEQ ID NO: 126;
the sense sequence comprises or is the polynucleotide sequence set forth in SEQ ID NO: 10, and the antisense sequence comprises or is the polynucleotide sequence set forth in SEQ ID NO: 129;
the sense sequence comprises or is the polynucleotide sequence set forth in SEQ ID NO: 11, and the antisense sequence comprises or is the polynucleotide sequence set forth in SEQ ID NO: 130;
the sense sequence comprises or is the polynucleotide sequence set forth in SEQ ID NO: 12, and the antisense sequence comprises or is the polynucleotide sequence set forth in SEQ ID NO: 131;
the sense sequence comprises or is the polynucleotide sequence set forth in SEQ ID NO: 17, and the antisense sequence comprises or is the polynucleotide sequence set forth in SEQ ID NO: 136;
the sense sequence comprises or is the polynucleotide sequence set forth in SEQ ID NO: 18, and the antisense sequence comprises or is the polynucleotide sequence set forth in SEQ ID NO: 137;
the sense sequence comprises or is the polynucleotide sequence set forth in SEQ ID NO: 19, and the antisense sequence comprises or is the polynucleotide sequence set forth in SEQ ID NO: 138;
the sense sequence comprises or is the polynucleotide sequence set forth in SEQ ID NO: 24, and the antisense sequence comprises or is the polynucleotide sequence set forth in SEQ ID NO: 143;
the sense sequence comprises or is the polynucleotide sequence set forth in SEQ ID NO: 30, and the antisense sequence comprises or is the polynucleotide sequence set forth in SEQ ID NO: 149;
the sense sequence comprises or is the polynucleotide sequence set forth in SEQ ID NO: 31, and the antisense sequence comprises or is the polynucleotide sequence set forth in SEQ ID NO: 150;
the sense sequence comprises or is the polynucleotide sequence set forth in SEQ ID NO: 81, and the antisense sequence comprises or is the polynucleotide sequence set forth in SEQ ID NO: 200;
the sense sequence comprises or is the polynucleotide sequence set forth in SEQ ID NO: 82, and the antisense sequence comprises or is the polynucleotide sequence set forth in SEQ ID NO: 201; or
the sense sequence comprises or is the polynucleotide sequence set forth in SEQ ID NO: 94, and the antisense sequence comprises or is the polynucleotide sequence set forth in SEQ ID NO: 213.

In some embodiments, the nucleic acid molecule is a dsRNA or shRNA. In some embodiments, the dsRNA is an siRNA. In some embodiments, one or more nucleotides in the nucleic acid molecule are chemically modified. In some embodiments, the chemical modification makes the nucleic acid molecule more stable in the cellular or in vivo environment. In some embodiments, the chemical modification prolongs the in vivo and/or in vitro half-life of the nucleic acid molecule. In some embodiments, the chemical modification comprises, or consists of, one or more modifications selected from the group consisting of:
locked nucleic acid modification, unlocked nucleic acid modification, 2'-methoxyethyl modification, 2'-O-methyl modification (2'-OMe), 2'-O-allyl modification, 2'-C-alkyl modification, 2'-C-allyl modification, 2'-fluoro modification (2'-F), 2'-deoxy modification (d), phosphorothioate modification (s), 2'-amino modification, morpholino modification, phosphoramidate modification, methylphosphonate modification, tetrahydropyran modification, 1,5-anhydrohexitol modification, 5'-vinyl phosphate modification, and cyclohexenyl modification.

In some embodiments, in the nucleic acid molecule, the nucleotides at positions 7, 9 to 12 of the sense strand comprise 2'-fluoro modifications, and/or the nucleotides at positions 2, 14 and 16 of the antisense strand comprise 2'-fluoro modifications. In some embodiments, in the nucleic acid molecule, the nucleotides at positions 7, 9 to 12 of the sense strand comprise 2'-fluoro modifications, and/or the nucleotides at positions 2, 14 and 16 of the antisense strand comprise 2'-fluoro modifications, and the remaining nucleotides comprise 2'-O-methyl modifications. In some embodiments, in the nucleic acid molecule, the nucleotides at positions 7, 9 to 12 of the sense strand are modified with 2'-fluoro, and/or the nucleotides at positions 2, 14 and 16 of the antisense strand are modified with 2'-fluor, the remaining nucleotides are modified with 2'-O-methyl, and the nucleic acid molecule comprises no additional modifications other than phosphorothioate modifications.

In some embodiments, in the nucleic acid molecule, the nucleotides at positions 7, 9 to 11 of the sense strand comprise 2'-fluoro modifications, and/or the nucleotides at positions 2, 14 and 16 of the antisense strand comprise 2'-fluoro modifications. In some embodiments, in the nucleic acid molecule, the nucleotides at positions 7, 9 to 11 of the sense strand comprise 2'-fluoro modifications, and/or the nucleotides at positions 2, 14 and 16 of the antisense strand comprise 2'-fluoro modifications, and the remaining nucleotides comprise 2'-O-methyl modifications. In some embodiments, in the nucleic acid molecule, the nucleotides at positions 7, 9 to 11 of the sense strand are modified with 2'-fluoro, and/or the nucleotides at positions 2, 14 and 16 of the antisense strand are modified with 2'-fluor, the remaining nucleotides are modified with 2'-O-methyl, and the nucleic acid molecule comprises no additional modifications other than phosphorothioate modifications.

In some embodiments, in the nucleic acid molecule, the nucleotides at positions 7, 9 to 11 of the sense strand comprise 2'-fluoro modifications, and/or the nucleotides at positions 2, 6, 14 and 16 of the antisense strand comprise 2'-fluoro modifications. In some embodiments, in the nucleic acid molecule, the nucleotides at positions 7, 9 to 11 of the sense strand comprise 2'-fluoro modifications, and/or the nucleotides at positions 2, 6, 14 and 16 of the antisense strand comprise 2'-fluoro modifications, and the remaining nucleotides comprise 2'-O-methyl modifications. In some embodiments, in the nucleic acid molecule, the nucleotides at positions 7, 9 to 11 of the sense strand are modified with 2'-fluoro, and/or the nucleotides at positions 2, 6, 14 and 16 of the antisense strand are modified with 2'-fluor, the remaining nucleotides are modified with 2'-O-methyl, and the nucleic acid molecule comprises no additional modifications other than phosphorothioate modifications.

In some embodiments, the nucleic acid molecule comprises a motif selected from the group consisting of:
(1) sense sequence: NmNmNmNmNmNmNfNmNfNfNfNfNmNmNmNmNmNmNmNmNm, antisense sequence: NmNfNmNmNmNmNmNmNmNmNmNmNmNfNmNfNmNmNmNmNmNmNm
(2) sense sequence: NmNmNmNmNmNmNfNmNfNfNfNmNmNmNmNmNmNmNmNmNm, antisense sequence: NmNfNmNmNmNmNmNmNmNmNmNmNmNfNmNfNmNmNmNmNmNmNm; and
(3) sense sequence: NmNmNmNmNmNmNfNmNfNfNfNmNmNmNmNmNmNmNmNmNm, antisense sequence: NmNfNmNmNmNfNmNmNmNmNmNmNmNfNmNfNmNmNmNmNmNmNm;
wherein in each sequence, N from 5' to 3' independently represents the nucleotide at the corresponding position in the sequence; Nm denotes a ribonucleotide modified with 2'-O-methyl, and Nf denotes a ribonucleotide modified with 2'-fluoro; it shall be understood that the present embodiment does not limit whether each nucleotide comprises any modifications other than those indicated herein.

In some embodiments, the above embodiments further comprise at least one phosphorothioate modification between the first and second nucleotides, and between the second and third nucleotides at the 5' end of the sense sequence; and comprise at least one phosphorothioate modification between the first and second nucleotides, between the second and third nucleotides, between the last and penultimate nucleotides, and between the penultimate and antepenultimate nucleotides at the 5' end of the antisense sequence.

In some embodiments, the nucleic acid molecule comprises a motif selected from any one of (1) to (9) below:
(1) sense sequence: NmsNmsNmNmNmNmNfNmNfNfNfNfNmNmNmNmNmNmNmNmNm, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNfNmNmNmNmNmNmNmNmNm, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(2) sense sequence: NmsNmsNmNmNmNmNfNmNfNfNfNmNmNmNmNmNmNmNmNmNm, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNm, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(3) sense sequence: NmsNmsNmNmNmNmNfNmNfNfNfNmNmNmNmNmNmNmNmNmNm, antisense sequence: NmsNfsNmNmNmNfNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNm, antisense sequence: NmsNfsNmNmNmNfNmNmNmNmNmNmNmNfNmNfNmNmNmNmNmsNmsNm, wherein each nucleotide comprises no modifications other than 2'-F, 2'-OMe and phosphorothioate modifications;
(4) sense sequence: NmsNmsNmNmNmNmNfNmNfNfNfNfNmNmNmNmNmNmNmsNmsNm, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmsNmsNm, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(5) sense sequence: NmsNmsNmNmNmNmNfNmNfNfNfNmNmNmNmNmNmNmNmsNmsNm, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmsNmsNm, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(6) sense sequence: NmsNmsNmNmNmNmNfNmNfNfNfNmNmNmNmNmNmNmNmsNmsNm, antisense sequence: NmsNfsNmNmNmNfNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmsNmsNm, antisense sequence: NmsNfsNmNmNmNfNmNmNmNmNmNmNmNfNmNfNmNmNmNmNmsNmsNm, wherein each nucleotide comprises no modifications other than 2'-F, 2'-OMe and phosphorothioate modifications;
(7) sense sequence: NmsNmsNmNmNmNmNfNmNfNfNfNfNmNmNmNmNmNmNmNmNms, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNms, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(8) sense sequence: NmsNmsNmNmNmNmNfNmNfNfNfNmNmNmNmNmNmNmNmNmNms, antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNms, antisense sequence: NmsNfsNmNmNmNmNmNmNmNmNmNmNmNfNmNfNmNmNmNmNmsNmsNm, wherein each nucleotide comprises no modifications other than 2'-F, 2'-OMe and phosphorothioate modifications;
(9) sense sequence: NmsNmsNmNmNmNmNfNmNfNfNfNmNmNmNmNmNmNmNmNmNms, antisense sequence: NmsNfsNmNmNmNfNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNms, antisense sequence: NmsNfsNmNmNmNfNmNmNmNmNmNmNmNfNmNfNmNmNmNmNmsNmsNm, wherein each nucleotide comprises no modifications other than 2'-F, 2'-OMe and phosphorothioate modifications.

In all motifs of the present application, Nm represents a ribonucleotide modified with 2'-O-methyl, Nf represents a ribonucleotide modified with 2'-fluoro, and s represents a phosphorothioate modification.

In some embodiments, the nucleic acid molecule is linked to an organ-targeting ligand, such as a liver-targeting ligand. In some embodiments, the nucleic acid molecule is linked to at least one asialoglycoprotein receptor (ASGPR) ligand. In some embodiments, the organ-targeting ligand is linked to the 5' end or 3' end of the sense sequence. In some embodiments, the ASGPR ligand is one or more GalNAc derivatives linked via a divalent or trivalent branched structure. In some embodiments, the GalNAc derivative comprises the following structure:

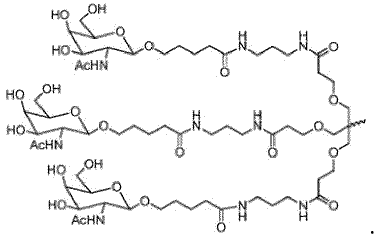

In some embodiments, the GalNAc derivative is L96, and the structure of the L96 is shown below:

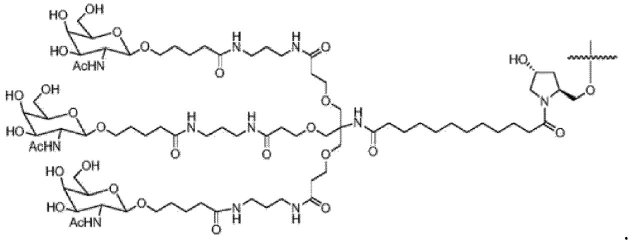

In some embodiments, the GalNAc derivative is ligand 1, and the structure of the ligand 1 is shown below: 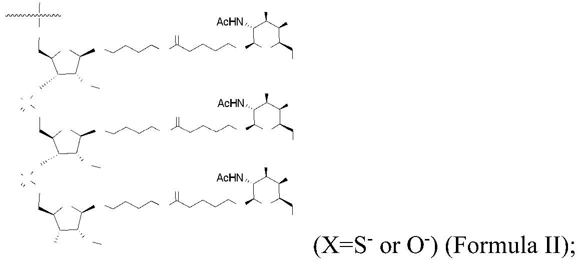 wherein 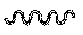 in formula I and formula II represents linkage to the 3' end of the sense sequence or antisense sequence of the nucleic acid molecule; preferably, the linkage is formed via a phosphodiester bond or a phosphorothioate diester bond.

More preferably, the nucleic acid molecule has a modification motif according to any one of (1) to (6):
(1) sense sequence: NmsNmsNmNmNmNmNfNmNfNfNfNfNmNmNmNmNmNmNmNmNm-L96; antisense strand: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNfNmNmNmNmNmNmNmNmNm-L96; antisense strand: NmsNfsNmNmNmNmNmNmNmNmNmNmNmNfNmNfNmNmNmNmNmsNmsNm; wherein each nucleotide comprises no modifications other than L96, 2'-F, 2'-OMe and phosphorothioate modifications;
(2) sense sequence: NmsNmsNmNmNmNmNfNmNfNfNfNfNmNmNmNmNmNmNmNmNms-ligand 1; antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNfNmNmNmNmNmNmNmNmNms-ligand 1, antisense sequence: NmsNfsNmNmNmNmNmNmNmNmNmNmNmNfNmNfNmNmNmNmNmsNmsNm; wherein each nucleotide comprises no modifications other than ligand-1, 2'-F, 2'-OMe and phosphorothioate modifications;
(3) sense sequence: NmsNmsNmNmNmNmNfNmNfNfNfNmNmNmNmNmNmNmNmNmNm-L96; antisense sequence: NmsNfsNmNmNmNfNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNm-L96; antisense sequence: NmsNfsNmNmNmNfNmNmNmNmNmNmNmNfNmNfNmNmNmNmNmsNmsNm; wherein each nucleotide comprises no modifications other than L96, 2'-F, 2'-OMe and phosphorothioate modifications;
(4) sense sequence: NmsNmsNmNmNmNmNfNmNfNfNfNmNmNmNmNmNmNmNmNmNms-ligand 1; antisense sequence: NmsNfsNmNmNmNfNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNms-ligand 1, antisense sequence: NmsNfsNmNmNmNfNmNmNmNmNmNmNmNfNmNfNmNmNmNmNmsNmsNm; wherein each nucleotide comprises no modifications other than ligand-1, 2'-F, 2'-OMe and phosphorothioate modifications;
(5) sense sequence: NmsNmsNmNmNmNmNfNmNfNfNfNmNmNmNmNmNmNmNmNmNm-L96; antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNm-L96; antisense sequence: NmsNfsNmNmNmNmNmNmNmNmNmNmNmNfNmNfNmNmNmNmNmsNmsNm; wherein each nucleotide comprises no modifications other than L96, 2'-F, 2'-OMe and phosphorothioate modifications;
(6) sense sequence: NmsNmsNmNmNmNmNfNmNfNfNfNmNmNmNmNmNmNmNmNmNms-ligand 1; antisense sequence: NmsNfsNmNmNmNmNmNmNmNmNmNmNmNfNmNfNmNmNmNmNmsNmsNm;
   in other embodiments: sense sequence: NmsNmsNmNmNmNmNfNmNfNfNfNmNmNmNmNmNmNmNmNmNms-ligand 1, antisense sequence: NmsNfsNmNmNmNmNmNmNmNmNmNmNmNfNmNfNmNmNmNmNmsNmsNm; wherein each nucleotide comprises no modifications other than ligand-1, 2'-F, 2'-OMe and phosphorothioate modifications.

In some embodiments, the nucleic acid molecule comprises a 5'-phosphate or 5' phosphate mimetic modification, such as a phosphate or phosphate mimetic at the 5' end of the antisense strand; preferably, the 5' phosphate mimetic modification is 5'-VP.

In some embodiments, the nucleic acid molecule has the following modification motifs:
GN-E20VP:
sense sequence: NmsNmsNmNmNmNmNfNmNfNfNfNfNmNmNmNmNmNmNmNmNms-ligand 1
antisense sequence: vp-NmsNfsNmNmNmNmNmNmNmNmNmNmNmNfNmNfNmNmNmNmNmsNmsNm;
in other embodiments, sense sequence: NmsNmsNmNmNmNmNfNmNfNfNfNfNmNmNmNmNmNmNmNmNms-ligand 1
antisense sequence: vp-NmsNfsNmNmNmNmNmNmNmNmNmNmNmNfNmNfNmNmNmNmNmsNmsNm; wherein each nucleotide comprises no modifications other than vp, ligand-1, 2'-F, 2'-OMe and phosphorothioate modifications.

In some embodiments, the nucleic acid molecule has the following modification motifs:
GN-E04VP:
sense sequence: NmsNmsNmNmNmNmNfNmNfNfNfNmNmNmNmNmNmNmNmNmNms-ligand 1
antisense sequence: vp-NmsNfsNmNmNmNfNmNmNmNmNmNmNmNfNmNfNmNmNmNmNmsNmsNm;
in other embodiments, sense sequence: NmsNmsNmNmNmNmNfNmNfNfNfNmNmNmNmNmNmNmNmNmNms-ligand 1
antisense sequence: vp-NmsNfsNmNmNmNfNmNmNmNmNmNmNmNfNmNfNmNmNmNmNmsNmsNm; wherein each nucleotide comprises no modifications other than vp, ligand-1, 2'-F, 2'-OMe and phosphorothioate modifications.

In some embodiments, the nucleic acid molecule has the following modification motifs:
GN-E05VP:
sense sequence: NmsNmsNmNmNmNmNfNmNfNfNfNmNmNmNmNmNmNmNmNmNms-ligand 1
antisense sequence: vp-NmsNfsNmNmNmNmNmNmNmNmNmNmNmNfNmNfNmNmNmNmNmsNmsNm;
in other embodiments, sense sequence: NmsNmsNmNmNmNmNfNmNfNfNfNmNmNmNmNmNmNmNmNmNms-ligand 1
antisense sequence: vp-NmsNfsNmNmNmNmNmNmNmNmNmNmNmNfNmNfNmNmNmNmNmsNmsNm; wherein each nucleotide comprises no modifications other than vp, ligand-1, 2'-F, 2'-OMe and phosphorothioate modifications.

In some embodiments, the nucleic acid molecule has the following modification motifs:
LN-E20VP:
sense sequence: NmsNmsNmNmNmNmNfNmNfNfNfNfNmNmNmNmNmNmNmNmNm-L96
antisense sequence: vp-NmsNfsNmNmNmNmNmNmNmNmNmNmNmNfNmNfNmNmNmNmNmsNmsNm;
in other embodiments, sense sequence: NmsNmsNmNmNmNmNfNmNfNfNfNfNmNmNmNmNmNmNmNmNm-L96
antisense sequence: vp-NmsNfsNmNmNmNmNmNmNmNmNmNmNmNfNmNfNmNmNmNmNmsNmsNm; wherein each nucleotide comprises no modifications other than vp, L96, 2'-F, 2'-OMe and phosphorothioate modifications.

In some embodiments, the nucleic acid molecule has the following modification motifs:
LN-E04VP:
sense sequence: NmsNmsNmNmNmNmNfNmNfNfNfNmNmNmNmNmNmNmNmNmNm-L96
antisense sequence: vp-NmsNfsNmNmNmNfNmNmNmNmNmNmNmNfNmNfNmNmNmNmNmsNmsNm;
in other embodiments, sense sequence: NmsNmsNmNmNmNmNfNmNfNfNfNmNmNmNmNmNmNmNmNmNm-L96
antisense sequence: vp-NmsNfsNmNmNmNfNmNmNmNmNmNmNmNfNmNfNmNmNmNmNmsNmsNm; wherein each nucleotide comprises no modifications other than vp, L96, 2'-F, 2'-OMe and phosphorothioate modifications.

In some embodiments, the nucleic acid molecule has the following modification motifs:
LN-E05VP:
sense sequence: NmsNmsNmNmNmNmNfNmNfNfNfNmNmNmNmNmNmNmNmNmNm-L96
antisense sequence: vp-NmsNfsNmNmNmNmNmNmNmNmNmNmNmNfNmNfNmNmNmNmNmsNmsNm;
in other embodiments, sense sequence: NmsNmsNmNmNmNmNfNmNfNfNfNmNmNmNmNmNmNmNmNmNm-L96
antisense sequence: vp-NmsNfsNmNmNmNmNmNmNmNmNmNmNmNfNmNfNmNmNmNmNmsNmsNm; wherein each nucleotide comprises no modifications other than vp, L96, 2'-F, 2'-OMe and phosphorothioate modifications.

In some embodiments, the naked sequences of the nucleic acid molecules specifically provided herein include the sense sequence: SEQ ID NO: 58 and the antisense sequence: SEQ ID NO: 177 (siRNA No. 50), which adopt any one of the modification patterns listed above.

In some embodiments, the naked sequences of the nucleic acid molecules specifically provided herein include the sense sequence: SEQ ID NO: 58 and the antisense sequence: SEQ ID NO: 177 (siRNA No. 50), which adopt the following modification patterns:
GN-E20:
   sense sequence: NmsNmsNmNmNmNmNfNmNfNfNfNfNmNmNmNmNmNmNmNmNms-ligand 1,
   antisense sequence: NmsNfsNmNmNmNmNmNmNmNmNmNmNmNfNmNfNmNmNmNmNmsNmsNm;
GN-E20VP:
   sense sequence: NmsNmsNmNmNmNmNfNmNfNfNfNfNmNmNmNmNmNmNmNmNms-ligand 1,
   antisense sequence: vp-NmsNfsNmNmNmNmNmNmNmNmNmNmNmNfNmNfNmNmNmNmNmsNmsNm;
GN-E04:
   sense sequence: NmsNmsNmNmNmNmNfNmNfNfNfNmNmNmNmNmNmNmNmNmNms-ligand 1,
   antisense sequence: NmsNfsNmNmNmNfNmNmNmNmNmNmNmNfNmNfNmNmNmNmNmsNmsNm;
GN-E04VP:
   sense sequence: NmsNmsNmNmNmNmNfNmNfNfNfNmNmNmNmNmNmNmNmNmNms-ligand 1,
   antisense sequence: vp-NmsNfsNmNmNmNfNmNmNmNmNmNmNmNfNmNfNmNmNmNmNmsNmsNm;
GN-E05:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm; or
GN-E05VP:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm.

In some embodiments, the naked sequences of the nucleic acid molecules specifically provided herein include the sense sequence: SEQ ID NO: 58 and the antisense sequence: SEQ ID NO: 177 (siRNA No. 50), which adopt the following modification patterns:
GN-E20:
   sense sequence: NmsNmsNmNmNmNmNfNmNfNfNfNfNmNmNmNmNmNmNmNmNms-ligand 1,
   antisense sequence: NmsNfsNmNmNmNmNmNmNmNmNmNmNmNfNmNfNmNmNmNmNmsNmsNm; wherein each nucleotide comprises no modifications other than ligand-1, 2'-F, 2'-OMe and phosphorothioate modifications;
GN-E20VP:
   sense sequence: NmsNmsNmNmNmNmNfNmNfNfNfNfNmNmNmNmNmNmNmNmNms-ligand 1,
   antisense sequence: vp-NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications;
GN-E04:
   sense sequence: NmsNmsNmNmNmNmNfNmNfNfNfNmNmNmNmNmNmNmNmNmNms-ligand 1,
   antisense sequence: NmsNfsNmNmNmNfNmNmNmNmNmNmNmNfNmNfNmNmNmNmNmsNmsNm, wherein each nucleotide comprises no modifications other than ligand-1, 2'-F, 2'-OMe and phosphorothioate modifications;
GN-E04VP:
   sense sequence: NmsNmsNmNmNmNmNfNmNfNfNfNmNmNmNmNmNmNmNmNmNms-ligand 1,
   antisense sequence: vp-NmsNfsNmNmNmNfNmNmNmNmNmNmNmNfNmNfNmNmNmNmNmsNmsNm, wherein each nucleotide comprises no modifications other than vp, ligand-1, 2'-F, 2'-OMe and phosphorothioate modifications;
GN-E05:
   sense sequence: NmsNmsNmNmNmNmNfNmNfNfNfNmNmNmNmNmNmNmNmNmNms-ligand 1,
   antisense sequence: NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications; or
GN-E05VP:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications.

In some embodiments, the naked sequences of the nucleic acid molecules provided herein include the sense sequence: SEQ ID NO: 34 and the antisense sequence: SEQ ID NO: 153 (siRNA No. 26'), which adopt any one of the modification patterns listed above.

In some embodiments, the naked sequences of the nucleic acid molecules provided herein include the sense sequence: SEQ ID NO: 34 and the antisense sequence: SEQ ID NO: 153 (siRNA No. 26'), which adopt the modification patterns shown below:
GN-E20:
   sense sequence: NmsNmsNmNmNmNmNfNmNfNfNfNfNmNmNmNmNmNmNmNmNms-ligand 1,
   antisense sequence: NmsNfsNmNmNmNmNmNmNmNmNmNmNmNfNmNfNmNmNmNmNmsNmsNm;
GN-E20VP:
   sense sequence: NmsNmsNmNmNmNmNfNmNfNfNfNfNmNmNmNmNmNmNmNmNms-ligand 1,
   antisense sequence: vp-NmsNfsNmNmNmNmNmNmNmNmNmNmNmNfNmNfNmNmNmNmNmsNmsNm;
GN-E04:
   sense sequence: NmsNmsNmNmNmNmNfNmNfNfNfNmNmNmNmNmNmNmNmNmNms-ligand 1,
   antisense sequence: NmsNfsNmNmNmNfNmNmNmNmNmNmNmNfNmNfNmNmNmNmNmsNmsNm;
GN-E04VP:
   sense sequence: NmsNmsNmNmNmNmNfNmNfNfNfNmNmNmNmNmNmNmNmNmNms-ligand 1,
   antisense sequence: vp-NmsNfsNmNmNmNfNmNmNmNmNmNmNmNfNmNfNmNmNmNmNmsNmsNm;
GN-E05:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm; or
GN-E05VP:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm.

In some embodiments, the naked sequences of the nucleic acid molecules specifically provided herein include the sense sequence: SEQ ID NO: 34 and the antisense sequence: SEQ ID NO: 153 (siRNA No. 26'), which adopt the following modification patterns:
GN-E20:
   sense sequence: NmsNmsNmNmNmNmNfNmNfNfNfNfNmNmNmNmNmNmNmNmNms-ligand 1,
   antisense sequence: NmsNfsNmNmNmNmNmNmNmNmNmNmNmNfNmNfNmNmNmNmNmsNmsNm; wherein each nucleotide comprises no modifications other than ligand-1, 2'-F, 2'-OMe and phosphorothioate modifications;
GN-E20VP:
   sense sequence: NmsNmsNmNmNmNmNfNmNfNfNfNfNmNmNmNmNmNmNmNmNms-ligand 1,
   antisense sequence: vp-NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications;
GN-E04:
   sense sequence: NmsNmsNmNmNmNmNfNmNfNfNfNmNmNmNmNmNmNmNmNmNms-ligand 1,
   antisense sequence: NmsNfsNmNmNmNfNmNmNmNmNmNmNmNfNmNfNmNmNmNmNmsNmsNm, wherein each nucleotide comprises no modifications other than ligand-1, 2'-F, 2'-OMe and phosphorothioate modifications;
GN-E04VP:
   sense sequence: NmsNmsNmNmNmNmNfNmNfNfNfNmNmNmNmNmNmNmNmNmNms-ligand 1,
   antisense sequence: vp-NmsNfsNmNmNmNfNmNmNmNmNmNmNmNfNmNfNmNmNmNmNmsNmsNm, wherein each nucleotide comprises no modifications other than vp, ligand-1, 2'-F, 2'-OMe and phosphorothioate modifications;
GN-E05:
   sense sequence: NmsNmsNmNmNmNmNfNmNfNfNfNmNmNmNmNmNmNmNmNmNms-ligand 1,
   antisense sequence: NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications; or
GN-E05VP:
   sense sequence: NmsNmsNmNmNmNmNfNmNfNfNfNmNmNmNmNmNmNmNmNmNms-ligand 1,
   antisense sequence: vp-NmsNfsNmNmNmNmNmNmNmNmNmNmNmNfNmNfNmNmNmNmNmsNmsNm, wherein each nucleotide comprises no modifications other than vp, ligand-1, 2'-F, 2'-OMe and phosphorothioate modifications.

In some embodiments, the naked sequences of the nucleic acid molecules specifically provided herein include the sense sequence: SEQ ID NO: 58 and the antisense sequence: SEQ ID NO: 177 (siRNA No. 50), which adopt the following modification patterns:
LN-E20:
   sense sequence: NmsNmsNmNmNmNmNfNmNfNfNfNfNmNmNmNmNmNmNmNmNm-L96,
   antisense sequence: NmsNfsNmNmNmNmNmNmNmNmNmNmNmNfNmNfNmNmNmNmNmsNmsNm;
LN-E20VP:
   sense sequence: NmsNmsNmNmNmNmNfNmNfNfNfNfNmNmNmNmNmNmNmNmNm-L96,
   antisense sequence: vp-NmsNfsNmNmNmNmNmNmNmNmNmNmNmNfNmNfNmNmNmNmNmsNmsNm;
LN-E04:
   sense sequence: NmsNmsNmNmNmNmNfNmNfNfNfNmNmNmNmNmNmNmNmNmNm-L96,
   antisense sequence: NmsNfsNmNmNmNfNmNmNmNmNmNmNmNfNmNfNmNmNmNmNmsNmsNm;
LN-E04VP:
   sense sequence: NmsNmsNmNmNmNmNfNmNfNfNfNmNmNmNmNmNmNmNmNmNm-L96,
   antisense sequence: vp-NmsNfsNmNmNmNfNmNmNmNmNmNmNmNfNmNfNmNmNmNmNmsNmsNm;
LN-E05:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm; or
LN-E05VP:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm.

In some embodiments, the naked sequences of the nucleic acid molecules specifically provided herein include the sense sequence: SEQ ID NO: 58 and the antisense sequence: SEQ ID NO: 177 (siRNA No. 50), which adopt the following modification patterns:
LN-E20:
   sense sequence: NmsNmsNmNmNmNmNfNmNfNfNfNfNmNmNmNmNmNmNmNmNm-L96,
   antisense sequence: NmsNfsNmNmNmNmNmNmNmNmNmNmNmNfNmNfNmNmNmNmNmsNmsNm, wherein each nucleotide comprises no modifications other than L96, 2'-F, 2'-OMe and phosphorothioate modifications;
LN-E20VP:
   sense sequence: NmsNmsNmNmNmNmNfNmNfNfNfNfNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications;
LN-E04:
   sense sequence: NmsNmsNmNmNmNmNfNmNfNfNfNmNmNmNmNmNmNmNmNmNm-L96,
   antisense sequence: NmsNfsNmNmNmNfNmNmNmNmNmNmNmNfNmNfNmNmNmNmNmsNmsNm, wherein each nucleotide comprises no modifications other than L96, 2'-F, 2'-OMe and phosphorothioate modifications;
LN-E04VP:
   sense sequence: NmsNmsNmNmNmNmNfNmNfNfNfNmNmNmNmNmNmNmNmNmNm-L96,
   antisense sequence: vp-NmsNfsNmNmNmNfNmNmNmNmNmNmNmNfNmNfNmNmNmNmNmsNmsNm, wherein each nucleotide comprises no modifications other than vp, L96, 2'-F, 2'-OMe and phosphorothioate modifications;
LN-E05:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications; or
LN-E05VP:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications.

In some embodiments, the naked sequences of the nucleic acid molecules provided herein include the sense sequence: SEQ ID NO: 34 and the antisense sequence: SEQ ID NO: 153 (siRNA No. 26'), which adopt the modification patterns shown below:
LN-E20:
   sense sequence: NmsNmsNmNmNmNmNfNmNfNfNfNfNmNmNmNmNmNmNmNmNm-L96,
   antisense sequence: NmsNfsNmNmNmNmNmNmNmNmNmNmNmNfNmNfNmNmNmNmNmsNmsNm;
LN-E20VP:
   sense sequence: NmsNmsNmNmNmNmNfNmNfNfNfNfNmNmNmNmNmNmNmNmNm-L96,
   antisense sequence: vp-NmsNfsNmNmNmNmNmNmNmNmNmNmNmNfNmNfNmNmNmNmNmsNmsNm;
LN-E04:
   sense sequence: NmsNmsNmNmNmNmNfNmNfNfNfNmNmNmNmNmNmNmNmNmNm-L96,
   antisense sequence: NmsNfsNmNmNmNfNmNmNmNmNmNmNmNfNmNfNmNmNmNmNmsNmsNm;
LN-E04VP:
   sense sequence: NmsNmsNmNmNmNmNfNmNfNfNfNmNmNmNmNmNmNmNmNmNm-L96,
   antisense sequence: vp-NmsNfsNmNmNmNfNmNmNmNmNmNmNmNfNmNfNmNmNmNmNmsNmsNm;
LN-E05:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm; or
LN-E05VP:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm.

In some embodiments, the naked sequences of the nucleic acid molecules specifically provided herein include the sense sequence: SEQ ID NO: 34 and the antisense sequence: SEQ ID NO: 153 (siRNA No. 26'), which adopt the following modification patterns:
LN-E20:
   sense sequence: NmsNmsNmNmNmNmNfNmNfNfNfNfNmNmNmNmNmNmNmNmNm-L96,
   antisense sequence: NmsNfsNmNmNmNmNmNmNmNmNmNmNmNfNmNfNmNmNmNmNmsNmsNm, wherein each nucleotide comprises no modifications other than L96, 2'-F, 2'-OMe and phosphorothioate modifications;
LN-E20VP:
   sense sequence: NmsNmsNmNmNmNmNfNmNfNfNfNfNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications;
LN-E04:
   sense sequence: NmsNmsNmNmNmNmNfNmNfNfNfNmNmNmNmNmNmNmNmNmNm-L96,
   antisense sequence: NmsNfsNmNmNmNfNmNmNmNmNmNmNmNfNmNfNmNmNmNmNmsNmsNm, wherein each nucleotide comprises no modifications other than L96, 2'-F, 2'-OMe and phosphorothioate modifications;
LN-E04VP:
   sense sequence: NmsNmsNmNmNmNmNfNmNfNfNfNmNmNmNmNmNmNmNmNmNm-L96,
   antisense sequence: vp-NmsNfsNmNmNmNfNmNmNmNmNmNmNmNfNmNfNmNmNmNmNmsNmsNm, wherein each nucleotide comprises no modifications other than vp, L96, 2'-F, 2'-OMe and phosphorothioate modifications;
LN-E05:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications; or
LN-E05VP:
   sense sequence: NmsNmsNmNmNmNmNfNmNfNfNfNmNmNmNmNmNmNmNmNmNm-L96,
   antisense sequence: vp-NmsNfsNmNmNmNmNmNmNmNmNmNmNmNfNmNfNmNmNmNmNmsNmsNm, wherein each nucleotide comprises no modifications other than vp, L96, 2'-F, 2'-OMe and phosphorothioate modifications.

In addition, the present application further provides a precursor of the aforementioned nucleic acid molecule, which may be an shRNA or dsRNA.

In a second aspect, the present application provides a second nucleic acid molecule. The second nucleic acid molecule can be transcribed into a dsRNA or shRNA precursor in cells, and the dsRNA or shRNA is the nucleic acid molecule according to the first aspect. In some embodiments, the second nucleic acid molecule is a circular or linear nucleic acid molecule. In some embodiments, the second nucleic acid molecule is a circular or linear plasmid. In some embodiments, the nucleic acid molecule is derived from an artificially constructed viral genome, including but not limited to lentiviral vectors or other retroviral vectors, adenoviral vectors, AAV vectors, poxviral vectors, baculoviral vectors and herpes simplex viral vectors. In some embodiments, the nucleic acid molecule is derived from a cellular genome, such as a nuclear genome, mitochondrial nucleic acid or cytoplasmic free nucleic acid.

In a third aspect, the present application further provides a nucleic acid delivery vehicle, which comprises the nucleic acid molecule according to the first aspect or the second nucleic acid molecule according to the second aspect. In some embodiments, the nucleic acid delivery vehicle is a liposome, lipid nanoparticle, other polymer, endosome, exosome or vesicle.

Also provided are viral particles comprising the second nucleic acid molecule according to the second aspect. In some embodiments, the viral particles are enveloped virus particles or togavirus particles. In some embodiments, the viral particles are pseudotyped viral particles. In some embodiments, the viral particles are AAV, baculovirus, poxvirus, herpesvirus, alphavirus, lentivirus or other retrovirus particles.

Also provided are cells comprising the second nucleic acid molecule according to the second aspect. In some embodiments, the cells are prokaryotic cells. In some embodiments, the cells are eukaryotic cells, such as stem cells, including hematopoietic stem cells, mesenchymal stem cells and the like.

The present application further provides a pharmaceutical composition, which comprises the nucleic acid molecule according to the first aspect or a salt thereof, and a pharmaceutically acceptable carrier or diluent. In some embodiments, the pharmaceutical composition is used for treating or preventing thromboembolic complications or coagulation disorders in a subject. Preferably, the thromboembolic complications are one or more selected from the group consisting of deep vein thrombosis, pulmonary embolism, myocardial infarction and stroke.

In a fourth aspect, the present application further provides the use of the nucleic acid according to the first aspect, the second nucleic acid according to the second aspect, the nucleic acid delivery vehicle according to the third aspect, as well as the aforementioned viral particles, the aforementioned cells and the aforementioned pharmaceutical compositions.

For example, the present application provides the use of the nucleic acid molecule according to the first aspect or a salt thereof, the second nucleic acid molecule according to the second aspect, the aforementioned nucleic acid delivery vehicle, the aforementioned viral particles or the aforementioned cells in the manufacture of a drug for preventing or treating thromboembolic complications or coagulation disorders in a subject. In some embodiments, the thromboembolic complications are preferably one or more selected from the group consisting of deep vein thrombosis, pulmonary embolism, myocardial infarction and stroke.

The present application further provides a method for treating or preventing thromboembolic complications or coagulation disorders in a subject, wherein the method comprises administering an effective amount of the nucleic acid molecule according to the first aspect or a salt thereof, the second nucleic acid molecule according to the second aspect, the aforementioned nucleic acid delivery vehicle, the aforementioned viral particles, the aforementioned cells or the aforementioned pharmaceutical composition to a subject in need thereof. In some embodiments, the thromboembolic complications are preferably one or more selected from the group consisting of deep vein thrombosis, pulmonary embolism, myocardial infarction and stroke.

Also provided is a compound for treating or preventing thromboembolic complications or coagulation disorders in a subject, which comprises the nucleic acid molecule according to the first aspect or a salt thereof, the second nucleic acid molecule according to the second aspect, the aforementioned nucleic acid delivery vehicle, the aforementioned viral particles, the aforementioned cells or the aforementioned pharmaceutical composition. In some embodiments, the thromboembolic complications are preferably one or more selected from the group consisting of deep vein thrombosis, pulmonary embolism, myocardial infarction and stroke.

The present application further provides the use of the nucleic acid molecule according to the first aspect or a salt thereof or the second nucleic acid molecule according to the second aspect for inhibiting the expression of factor 11.

The present application further provides the use of the nucleic acid molecule according to the first aspect or a salt thereof or the second nucleic acid molecule according to the second aspect in the manufacture of a drug for inhibiting FXI expression in a subject.

The present application further provides a compound for inhibiting FXI expression in a subject, wherein the compound comprises the nucleic acid molecule according to the first aspect or a salt thereof, as well as a pharmaceutically acceptable carrier or diluent. The preferred embodiments of the present application have been described in detail above, but the present application is not limited thereto. Various simple modifications may be made to the technical solutions of the present application within the scope of its technical concept, including combining individual technical features in any other suitable manner. All such simple modifications and combinations shall be deemed part of the disclosure of the present application and fall within its protection scope. The aspects and embodiments described herein include those embodied by the terms "comprising", "consisting of" and "consisting essentially of".

### BRIEF DESCRIPTION OF THE DRAWINGS

FIG. 1 shows the high-throughput screening results of naked dsRNA nucleic acid molecules (5 nM) in HepG2 cells.
FIG. 2 shows the relative levels of FXI mRNA in HepG2 cells upon administration of naked nucleic acid molecules of candidate dsRNA or positive control dsRNA at concentrations of 5 nM and 1 nM, respectively.
FIG. 3 shows the inhibition rate of FXI mRNA in HepG2 cells by dsRNA modified with modification E at a concentration of 1 nM.
FIG. 4 shows the inhibition rates of FXI mRNA by GalNAc-conjugated dsRNA at different concentrations.
FIG. 5 shows the in vivo inhibition results of target protein after administration of different GalNAc-conjugated dsRNAs.
FIG. 6 shows the comparison results of different GalNAc after in vivo administration.
FIG. 7 shows the comparison of in vitro cellular IC50 values of dsRNAs with different modifications.
FIG. 8 shows the comparison results of dsRNAs with different modification motifs after in vivo administration.

### DETAILED DESCRIPTION

The present invention provides a nucleic acid molecule capable of triggering cleavage of RNA transcripts of the coagulation factor FXI (or FX11) gene mediated by the RNA-induced silencing complex (RISC), as well as a second nucleic acid molecule capable of transcribing the nucleic acid molecule. Also provided are delivery vehicles for the nucleic acid molecule and the second nucleic acid molecule, viruses or cells capable of transcribing the nucleic acid molecule, and uses of the nucleic acid molecule and the second nucleic acid molecule.

### Terms

For the purposes of this specification, the following definitions will apply, and where appropriate, terms used in the singular form will also include the plural form, and vice versa. Unless otherwise defined, all technical and scientific terms used herein shall have the same meanings as commonly understood by those skilled in the art to which the present invention pertains. All technical and patent publications cited herein are incorporated by reference in their entirety.

As used herein, "dsRNA" refers to double-stranded RNA. Since siRNA is double-stranded RNA, the term "dsRNA" encompasses siRNA. dsRNA also includes double-stranded RNA longer than siRNA. A dsRNA longer than siRNA means that its sense strand, antisense strand, or both sense and antisense strands are longer than those of siRNA. Generally, after entering cells, double-stranded RNAs longer than the siRNA sequence they contain are cleaved into siRNA by Dicer, a type III endonuclease. In some embodiments, each strand of the dsRNA independently has a length of 15 to 30 nt (as used herein, "nt" stands for nucleotide). After "siRNA" is incorporated into the RNA-induced silencing complex (RISC), one or more helicases within RISC unwind the siRNA duplex. Upon binding to target mRNA complementary to the antisense strand of the siRNA, one or more endonucleases in RISC cleave the target, thereby inducing gene silencing. Typically, most nucleotides of each strand of a dsRNA molecule are ribonucleotides. However, one or both strands may contain one or more non-ribonucleotides, such as deoxyribonucleotides and/or non-natural nucleotides. In some embodiments, the dsRNA molecule comprises no non-natural nucleotides. In some embodiments, every nucleotide of the dsRNA is a ribonucleotide. As used herein, a dsRNA may or may not comprise one or more chemically modified nucleotides.

The terms "FXI", "coagulation factor FXI" and "factor 11" are used interchangeably, and are also referred to as FXI or PTA in the art. FXI can be an FXI derived from mammals. In some embodiments, the FXI is a primate-derived FXI. In some embodiments, the FXI is a human-derived FXI. In some embodiments, FXI is an FXI derived from cynomolgus monkeys. As used herein, the term "FXI gene mRNA" refers to mRNA that encodes factor 11 protein. It is transcribed from the DNA of the FXI gene, and may be mature mRNA or mRNA precursor (pre-mRNA), and therefore may contain or be free of introns. Since minor nucleotide mutations may exist in the factor 11 gene among different individuals, unless otherwise specified, the factor 11 gene mRNA sequences described herein cover all mRNA sequences transcribed from mutants of the factor 11 gene. The human FXI gene mRNA sequence can be found, for example, in the gene bank accession number GI: 1732746318 (NM_000128.4). The rhesus monkey FXI gene mRNA sequence can be found, for example, in the gene bank accession number GI: 1622942384 (XM_015139652.2). The mRNA sequence of the cynomolgus monkey FXI gene can be found, for example, in the Genebank accession number GI: 2161917139 (XM_005556483.3). The mouse FXI gene mRNA sequence can be found, for example, in the Genebank accession number GI: 2293430447 (NM_028066.3). The rat FXI gene mRNA sequence can be found, for example, in the Genebank accession number GI: 2293332621 (NM_001411666.1). Additional FXI gene mRNA sequences can be readily retrieved from publicly accessible databases such as GenBANK. Unless otherwise specified, SEQ ID NO: 244 (i.e., NM_000128.4) is adopted as the reference sequence for FXI gene mRNA. That is, the numbering of each nucleotide position in the FXI gene mRNA corresponds to the nucleotide numbering in the 5' to 3' direction of the reference sequence SEQ ID NO: 244. As used herein, the term "reference sequence" means a standard sequence for homologous sequence alignment, which is used to define nucleotide positions within homologous polynucleotides or polynucleotide sequences. For example, the phrase "the base position numbering of the FXI gene mRNA sequence follows that of the corresponding bases in the reference sequence SEQ ID NO: 244" means that gaps are inserted or nucleotides are deleted in the FXI gene mRNA sequence to maximize base matches between the FXI gene mRNA sequence and the reference sequence. The nucleotides of the reference sequence are numbered consecutively starting from the first nucleotide at the 5' end. Nucleotides in the FXI gene mRNA sequence that align with those in the reference sequence are assigned identical position numbers.

As used herein, the term "complementary" in reference to nucleic acids means that one nucleic acid is capable of forming hydrogen bonds with another nucleic acid via traditional Watson-Crick base pairing. Percent complementarity refers to the percentage of nucleotides in the shorter of two nucleic acid molecules that can form hydrogen bonds (i.e., Watson-Crick base pairs) with the other nucleic acid molecule, relative to the total nucleotides of the shorter nucleic acid molecule. For example, approximately 5, 6, 7, 8, 9 and 10 out of 10 nucleotides correspond to about 50%, 60%, 70%, 80%, 90% and 100% complementarity, respectively. "Fully complementary" means that all contiguous residues of a nucleic acid sequence form hydrogen bonds with an equal number of contiguous residues in a second nucleic acid sequence. As used herein, "substantially complementary" means a complementarity level of at least any one of 70%, 75%, 80%, 85%, 90%, 95%, 96%, 97%, 98%, 99% or 100% over a region of about 40, 50, 60, 70, 80, 100, 150, 200, 250 or more nucleotides, or refers to two nucleic acids that hybridize under stringent conditions. For a single base or a single nucleotide, a base pair formed between A and T or U, and between C and G or I in accordance with Watson-Crick base pairing rules is defined as complementary, paired or matched, and vice versa. All other base pairings are deemed non-complementary.

As used herein, "hybridization" of nucleic acids refers to a reaction in which one or more polynucleotides react to form a complex stabilized by hydrogen bonds between nucleotide residues. Hydrogen bonding can occur through Watson-Crick base pairing, Hoogsteen binding, or any other sequence-specific mechanism. Such complexes may include two strands forming a duplex structure, three or more strands forming a multi-strand complex, a single self-hybridizing strand, or a combination thereof.

In the present application, the phrase that two polynucleotide sequences or two nucleic acid strands "hybridize at the maximum complementarity" means a hybridization pattern in which the maximum number of nucleotides of the two strands form pairs via hydrogen bonds. "Hybridization at the maximum complementarity" may permit one or more mismatches, as well as one or more bulges in one or both strands. However, in some embodiments, the two strands that "hybridize at the maximum complementarity" contain no mismatches or bulges after hybridization. As used herein, the term "complementary region" refers to all base pairs formed by hydrogen bonding, extending from the first hydrogen-bonded base pair at the 5' end to the last hydrogen-bonded base pair after the two strands hybridize. A complementary region formed by two hybridized strands may be continuous or discontinuous. "Complementary region 1" as used herein specifically refers to the complementary region composed of base pairs formed by complementary base pairing between the antisense sequence and the FXI gene mRNA when the antisense sequence hybridizes with the FXI gene mRNA at the maximum complementarity. "Complementary region 2" refers to the complementary region composed of base pairs formed by complementary base pairing between the antisense sequence and the sense sequence when the antisense sequence hybridizes with the sense sequence at the maximum complementarity. Since both complementary region 1 and complementary region 2 involve the antisense sequence, the antisense sequence portions of the complementary region 1 and complementary region 2 generally share partial or complete nucleotide composition identity. In the present application, the designations of antisense sequence and sense sequence are defined relative to a third sequence that is complementary to one of the two sequences. For example, for "a nucleic acid molecule that inhibits the expression of factor 11 (FXI) gene in cells via RNAi", its antisense sequence and sense sequence are defined with reference to the third sequence, i.e., the FXI gene mRNA sequence. Specifically, the antisense sequence refers to the sequence of the nucleic acid molecule that forms a complementary region with the FXI gene mRNA sequence; the sense sequence refers to the sequence of the nucleic acid molecule that has at least 10 consecutive identical nucleotides relative to the FXI gene mRNA sequence. In addition, the 5' end of any complementary region herein refers to the position of the nucleotide or base pair within the complementary region that is closest to the 5' end of the sense strand or the aforesaid third sequence. The 5' end of the complementary region refers to the side or extremity of the complementary region that is relatively proximal to the 5' end of the sense strand or the aforesaid third sequence. Similarly, the 3' end of any complementary region herein refers to the position of the nucleotide or base pair within the complementary region that is closest to the 3' end of the sense strand or the aforesaid third sequence. The 3' end of the complementary region refers to the side or extremity of the complementary region that is relatively proximal to the 3' end of the sense strand or the aforesaid third sequence. In the present application, when describing the terms "proximal", "close" or "distant" with respect to nucleotide positions on the same sequence or nucleic acid strand, such terms refer to a smaller or larger number of intervening nucleotides between the two positions.

As used herein, the term "overhang nucleotide" is referred with respect to the complementary region 2, and refers to nucleotides located outside the complementary region 2 in the nucleotides of the sense sequence and/or the antisense sequence after the sense sequence and the antisense sequence are hybridized at maximum complementarity. In some embodiments, the overhang nucleotides are present only on the sense sequence. In some embodiments, the overhang nucleotides are present only on the antisense sequence. In some embodiments, the overhang nucleotides are present on both the sense sequence and the antisense sequence. In some embodiments, the overhang nucleotides are located only on the 5' side of the complementary region 2. In some embodiments, the overhang nucleotides are located only on the 5' side of the complementary region 2 of the sense strand. In some embodiments, the overhang nucleotides are located only on the 5' side of the complementary region 2 of the antisense strand. In some embodiments, the overhang nucleotides are located only on the 3' side of the complementary region 2. In some embodiments, the overhang nucleotides are located only on the 3' side of the complementary region 2 of the sense strand. In some embodiments, the overhang nucleotides are located only on the 3' side of the complementary region 2 of the antisense strand. In some embodiments, the number of overhang nucleotides on the same side (i.e., the 5' end or the 3' end) of the sense sequence or the antisense sequence does not exceed two (namely 1 or 2). In the present application, the expressions "5' side" and "3' side" are used to describe the relative positional relationship between two sequence segments, two nucleotides, or one nucleotide and one sequence segment in the same polynucleotide sequence. The "5' end" refers to the end of the polynucleotide sequence bearing a free 5' phosphate group or a free 5' hydroxyl group. The "3' end" refers to the end of the polynucleotide sequence bearing a free 3' hydroxyl group or a free 3' phosphate group. A sequence or nucleotide on the 3' side of a given sequence in a nucleic acid strand is closer to the 3' end of the entire nucleic acid strand than the given sequence. For example, the phrase "located on the 5' side of the complementary region 2" with respect to a certain sequence or one or more nucleotides means that "such sequence or nucleotide(s)" is/are closer to the 5' end of the polynucleotide sequence (e.g., the antisense sequence or the sense sequence) where they resides, relative to "the complementary region 2".

The term "nucleotide", in addition to referring to naturally occurring ribonucleotide or deoxyribonucleotide monomers, shall also be construed herein as including related structural variants thereof, such as derivatives and analogs that are functionally equivalent in the specific context of use, unless the context clearly dictates otherwise. For example, "nucleotide" refers to deoxyribonucleotide or ribonucleotide. A nucleotide may be a canonical nucleotide (i.e., adenosine, guanosine, cytidine, thymidine and uridine), a nucleotide isomer or a nucleotide analog. A nucleotide analog refers to a nucleotide with a modified purine or pyrimidine base or a modified ribose moiety. Nucleotide analogs can be naturally occurring nucleotides (e.g., inosine, pseudouridine, etc.) or non-naturally occurring nucleotides. Non-limiting examples of modifications on the sugar or base moiety of a nucleotide include the addition (or removal) of acetyl, amino, carboxyl, carboxymethyl, hydroxyl, methyl, phosphoryl and thiol groups, as well as the substitution of carbon and nitrogen atoms in bases with other atoms (e.g., 7-deazapurine). Nucleotide analogs further include dideoxynucleotides, 2'-O-methyl nucleotides, locked nucleic acids (LNA), peptide nucleic acids (PNA) and morpholino oligonucleotides (morpholino). In some embodiments, the "nucleotide" as used herein excludes non-naturally occurring nucleotides with modified bases. In some embodiments, the "nucleotide" as used herein excludes any nucleotides with modified bases. As used herein, "G", "C", "A", "T" and "U" generally represent nucleotides with guanine, cytosine, adenine, thymine and uracil as their respective bases. Unless otherwise specified, "G", "C", "A", "T" and "U" indicate nucleotides without limitation on the modifications they contain, that is, they can be used to represent natural nucleotides or non-natural nucleotides. Such non-naturally occurring nucleotides may have modified ribose and/or modified bases, provided that the bases are still capable of forming complementary hydrogen bonds with their natural pairing counterparts in accordance with Watson-Crick base pairing rules. However, in the context of RNA and in RNA sequences, unless otherwise specified, "T" refers to uridine or uracil. It shall be understood that in any context relating to nucleotide sequences herein, "nucleotide", "nucleotide residue" and "base" are used interchangeably. The number of base pairs is expressed in bp, where one bp equals one base pair. The number of nucleotides is expressed in nt, where one nt equals one nucleotide.

As used herein, the term "internucleotide linkage" refers to a chemical bond (or linking group) between two adjacent nucleotides, or between a nucleotide and a ligand. Unless otherwise specified, such chemical bonds are phosphoester bonds or phosphodiester bonds. Where the letter "s" is marked in a modification motif to indicate a phosphorothioate modification, only the internucleotide linkage at the position labeled "s" shall be a phosphorothioate bond; all other unmarked internucleotide linkages shall be phosphoester bonds. Examples are as follows:
(1) sense strand: NmNmNmNmNmNmNfNmNfNdNfNmNmNmNmNmNmNmNmNmNm, antisense strand: NmNfNmNmNdNmNdNmNmNmNmNmNmNmNfNmNfNmNmNmNmNmNmNm;
   all internucleotide linkages in (1) may be either phosphoester bonds or phosphorothioate bonds;
   however, if it is as follows: sense chain: CmsAmsGmAmGmUmUfAmUfCdGfAmGmGmCmAmCmAmUmUmAms-ligand 1,
   antisense strand: vp-UmsAfsAmUmGdUmGdCmCmUmCmGmAmUfAmAfCmUmCmUmGmsGmsCm, since "s" is specifically marked here, except that the internucleotide linkages at the positions specified below are phosphorothioate bonds, and all other internucleotide linkages are phosphoester bonds: the first and second internucleotide linkages starting from the 5' end of the sense strand; the internucleotide linkage between the 3' end nucleotide of the sense strand and ligand 1; the first and second internucleotide linkages starting from the 5' end of the antisense strand; and the first and second internucleotide linkages starting from the 3' end of the antisense strand. The first internucleotide linkage means the chemical bond between the first and second nucleotides starting from an end (e.g., the 5' end or 3' end). The second internucleotide linkage means the chemical bond between the second and third nucleotides starting from an end (e.g., the 5' end or 3' end), and so on. The nth internucleotide linkage means the chemical bond between the nth nucleotide and the (n+1)th nucleotide or ligand starting from an end (e.g., the 5' end or 3' end), and so forth.

As used herein, "overhang", "sticky end" and "overhanging sequence" are used interchangeably. They refer to one or more unpaired nucleotides extending beyond the duplex region at the end of a strand. Nucleotide overhangs are typically formed when the 3' end of one strand extends beyond the 5' end of the other strand, or when the 5' end of one strand extends beyond the 3' end of the other strand. The length of a nucleotide overhang is generally in the range of 1 to 6 nucleotides, 1 to 5 nucleotides, 1 to 4 nucleotides, 1 to 3 nucleotides, 2 to 6 nucleotides, 2 to 5 nucleotides, or 2 to 4 nucleotides. In some embodiments, the nucleotide overhangs comprise 1, 2, 3, 4, 5, or 6 nucleotides. In one particular embodiment, the nucleotide overhang comprises 1 to 4 nucleotides. In some embodiments, the nucleotide overhang comprises 2 nucleotides. In some other embodiments, the nucleotide overhang comprises a single nucleotide.

The nucleotides in an overhang may be ribonucleotides or modified nucleotides as described herein. In some embodiments, the nucleotides in the overhang are 2'-modified nucleotides (e.g., 2'-fluoro modified nucleotides, 2'-O-methyl modified nucleotides), deoxyribonucleotides, inverted nucleotides (e.g., inverted abasic nucleotides, inverted deoxyribonucleotides), or combinations thereof. For example, in one embodiment, the nucleotides in the overhang are deoxyribonucleotides, such as deoxythymidine. In another embodiment, the nucleotides in the overhang are 2'-O-methyl modified nucleotides, 2'-fluoro modified nucleotides, 2'-methoxyethyl modified nucleotides, or combinations thereof. In other embodiments, the overhang comprises 5'-uridine-uridine-3' (5'-UU-3') dinucleotide. In such embodiments, the UU dinucleotide may comprise a ribonucleotide or a modified nucleotide, such as a 2'-modified nucleotide. In other embodiments, the overhang comprises 5'-deoxythymidine-deoxythymidine-3' (5'-dTdT-3') dinucleotide. Where a nucleotide overhang is present on the antisense strand, the nucleotides of the overhang may be complementary to the target gene sequence, form mismatches with the target gene sequence, or contain other sequences (e.g., polypyrimidine or polypurine sequences such as UU, TT, AA, GG and the like).

A nucleotide overhang may be located at the 5' end or 3' end of one or both strands. For example, in one embodiment, the RNA molecule comprises nucleotide overhangs at both the 5' end and 3' end of the antisense strand. In another embodiment, the RNA molecule has nucleotide overhangs at both the 5' end and 3' end of the sense strand. In some embodiments, the RNA molecule has nucleotide overhangs at the 5' end of the sense strand and the 5' end of the antisense strand. In other embodiments, the RNA molecule has nucleotide overhangs at the 3' end of the sense strand and the 3' end of the antisense strand.

An RNA molecule may have a nucleotide overhang at one end of a double-stranded RNA molecule and a blunt end at the other end. "Blunt end" means that the sense strand and the antisense strand are fully base-paired at their molecular termini, with no unpaired nucleotides extending beyond the duplex region. In some embodiments, the RNA molecule has a nucleotide overhang at the 3' end of the sense strand, and blunt ends at the 5' end of the sense strand and the 3' end of the antisense strand. In other embodiments, the RNA molecule has a nucleotide overhang at the 3' end of the antisense strand, and blunt ends at the 5' end of the antisense strand and the 3' end of the sense strand. In some embodiments, the RNA molecule has blunt ends at both ends of the double-stranded RNA molecule. In such embodiments, the sense strand and the antisense strand are of equal length, and the length of the duplex region is identical to that of the sense strand and the antisense strand (i.e., the molecule is double-stranded along its entire length). As used herein, "GalNAc" or "N-acetylgalactosamine" refers to 2-(acetylamino)-2-deoxy-D-galactopyranose. Unless otherwise specified, the terms "GalNAc" and "N-acetylgalactosamine" encompass both the β-form: 2-(acetylamino)-2-deoxy-β-D-galactopyranose and the α-form: 2-(acetylamino)-2-deoxy-α-D-galactopyranose. Preferably, the GalNAc compound used herein is the β-form, namely 2-(acetylamino)-2-deoxy-β-D-galactopyranose.

As used herein, "modification VP" or "E-VP" refers to a 5'-phosphate mimic modification located on the antisense strand. Its specific structure is as follows: 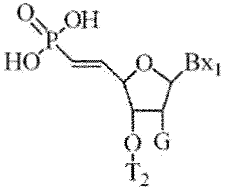 wherein:
Bx1 represents uracil, thymine, cytosine, 5-methylcytosine, adenine or guanine;
T₂ denotes a phosphodiester or phosphorothioate internucleotide linkage that connects the above compound to the oligonucleotide strand; G represents halogen, OCH₃, OCF₃, OCH₂CH₃, OCH₂CF₃, OCH₂-CH=CH₂, O(CH₂)₂-OCH₃, O(CH₂)₂-O(CH₂)₂-N(CH₃)₂, OCH₂C(=O)-N(H)CH₃, OCH₂C(=O)-N(H)-(CH₂)₂-N(CH₃)₂ or OCH₂-N(H)-C(=NH)NH₂.

As used herein, "3' end" specifically refers to the position of the first nucleotide or the first base pair at the 3' end of a single-stranded nucleotide sequence or a double-stranded polynucleotide. The term "5' end" specifically refers to the position of the first nucleotide or the first base pair at the 5' end of a single-stranded nucleotide sequence or a double-stranded polynucleotide.

As used herein, the term "nucleic acid molecule" refers to any molecule comprising a nucleotide sequence formed by more than two nucleotides linked via phosphoester bonds or modified phosphoester bonds (such as phosphorothioate bonds).

As used herein, the term "nucleotide sequence" means a polynucleotide strand consisting of nucleotides arranged in a specific order. Such a polynucleotide strand may constitute an entire nucleic acid molecule or a fragment of one strand of a nucleic acid molecule. Accordingly, a "nucleotide sequence" may be represented as a definite polynucleotide sequence composed of various nucleotides (e.g., ATCG), such as any one of SEQ ID NO: 1 to 100. It may also refer to nucleotides spanning from one position to another in a given sequence, for example, "nucleotides at positions 363 to 382" in the "FXI gene mRNA sequence". As used herein, unless otherwise specified, for any sequence or nucleic acid molecule comprising "nucleotides from one position to another in a given sequence", the arrangement order of the nucleotides from one position to another is identical to that of the aforesaid nucleotides in the given sequence. Without limitation, a "nucleotide sequence" may be RNA, DNA, or a hybrid molecule of RNA and DNA, and may also incorporate non-naturally occurring nucleotides or artificially modified nucleotides.

In addition, REL or relative expression level shown in the drawings of the present application refers to the relative expression level of mRNA. In the context of sequences, N denotes a ribonucleotide, dN denotes a deoxyribonucleotide (DNA), Nm denotes a 2'-O-Me modified nucleotide or a 2'-O-methyl modified nucleotide; Nf denotes a 2'-F modified ribonucleotide or a 2'-fluoro modified ribonucleotide; s denotes a phosphorothioate modification, namely a 5'-thio modified phosphate. When present between two nucleotides or between a nucleotide and a ligand, this modification replaces the phosphoester bond between native nucleotides and is referred to as a phosphorothioate bond. As used herein, unless otherwise specified, "phosphorothioate bond" and "phosphorothioate diester bond" are used interchangeably. Similarly, "phosphoester bond" and "phosphodiester bond" are used interchangeably.

As used herein, the term "about" refers to the customary margin of error for respective values as commonly known to those skilled in the art. References to a value or parameter preceded by "about" include embodiments directed to the value or parameter per se. As used herein, when the term "about" precedes a numerical value, it means a range within 10% above or below such value. For example, "about 100" encompasses the range from 90 to 110.

As used herein, the singular forms "a", "an" and "the" include plural referents unless the context clearly dictates otherwise.

Unless otherwise defined herein, all technical and scientific terms used herein have the same meaning as commonly understood by one of ordinary skill in the art to which the present invention belongs.

It is to be understood that the present application covers all aspects, embodiments described herein, as well as combinations of the aforesaid aspects and/or embodiments. The foregoing description and the subsequent examples are intended to illustrate, rather than limit, the scope of the present application. Various simple modifications may be made to the technical solutions of the present application within the scope of its technical concept, including combining individual technical features in any other suitable manner. All such simple modifications and combinations shall be deemed part of the disclosure of the present application and fall within its protection scope.

Unless otherwise indicated, the practice of the present application employs conventional techniques of cell biology, cell culture, molecular biology, transgenics, microbiology, recombinant DNA technology and immunology. Such conventional techniques are well documented in the prior art.

It is to be understood that the present application covers all aspects, embodiments described herein, as well as combinations of the aforesaid aspects and/or embodiments. The foregoing description and the subsequent examples are intended to illustrate, rather than limit, the scope of the present application. Other aspects, improvements and modifications falling within the scope of the present application will be apparent to those skilled in the art. Accordingly, a person of ordinary skill in the art will recognize that the scope of the present application also covers such improvements and modifications to the aforesaid aspects and embodiments.

### EXAMPLES

### Example 1: Sequence design and synthesis of dsRNA

In Examples 1 to 7 of the present application, none of the nucleotides comprise modifications not specified herein. That is, all nucleotide sequences used in Examples 1 to 7 of the present application are naked sequences, or only comprise modifications as indicated in their respective modification motifs.

### 1.1 Design of dsRNA

Based on the human FXI gene (Gene ID, 2160) mRNA sequence (NM_000128.4), multiple FXI dsRNAs were designed at different sites. All designed individual dsRNA sequences were compared with all other non-target gene sequences using sequence similarity software and showed the lowest homology. Details are listed in the Sequence Listing at the end of this document. For modified dsRNAs: if the first nucleotide at the 3' end of the sense strand is neither A nor U, the original nucleotide is replaced with uracil ribonucleotide. If the first nucleotide at the 5' end of the antisense sequence is neither A nor U, the original nucleotide is replaced with adenine ribonucleotide. The original nucleic acid sequence is changed from n to n' for detection. For instance, the nucleic acid sequences of E3, E4, E9, E14, E16, E24, E26, E51, E52, E69 and E70 correspond to the dsRNAs numbered 3', 4', 9', 14', 16', 24', 26', 51', 52', 69' and 70' respectively in the Sequence Listing at the end of this document.

In the present application, the ASO sequence of ISIS-416858 disclosed in the patent of ionis (Patent No.: CN 109797150 A) is adopted. We also select the top four sequences with the highest inhibition activity against the FXI gene disclosed in Patent No. CN113227376A (naked sequences designated RB2, RB4, RB6 and RB7, and modified sequences designated ERB2, ERB4, ERB6 and ERB7, and these naked and modified sequences correspond to the ligand-free sequences of L10-siFXIa1M1SP, L10-siFXIc1M1SP, L10-siFXIe1M1SP and L10-siFXIg1M1SP as recorded in the aforesaid patent). Furthermore, the top three sequences with the highest inhibition activity against the FXI gene disclosed in Patent No. WO 2022028457A1 are chosen as positive control sequences for the FXI gene (their naked sequences are named TJ632, TJ645 and TJ772, and the modified sequences are named ETJ632, ETJ645 and ETJ772, which correspond to the ligand-free sequences of TRD0632, TRD0645 and TRD0772 specified in the corresponding patent respectively). The naked sequences of the aforementioned dsRNA sequence and positive control sequence are shown in the Sequence Listing at the end of this document.

### 1.2 Synthesis and purification of dsRNA and conjugates thereof

dsRNA was synthesized using chemically modified (e.g., 2'-methoxy modified, 2'-fluoro modified or thio modified) or unmodified ribonucleotides at a theoretical synthesis scale of 1 µmol. All oligonucleotides were prepared on an LK-192X synthesizer (Jiangsu Lingkun Biotechnology Co., Ltd.) by adopting a common 1 µmol Frit support (1000 Å = 100 nm, Biocomma) or a CPG support loaded with protecting group-bearing GalNAc derivative L96 (Asymchem). In accordance with sequence requirements, all corresponding nucleoside phosphoramidite monomers were diluted with anhydrous acetonitrile at a ratio of 1:40 (g/mL). The coupling reaction was performed twice with a coupling time of 3 min for each run. Deprotection was performed using 3% TCA, activation was performed using 0.3 M benzylthiotetrazole in acetonitrile, and capping and oxidation were performed using CAPA/CAPB and 50 mM I2 solution, respectively. After the trityl-off synthesis, the solid support was transferred to a 2 mL centrifuge tube, 1.2 mL of ammonia was added, and the tube was heated in an oven at 65 °C for 3 h to remove the protecting group. The mixture was then cooled to room temperature and concentrated under vacuum for 30 min. The resulting solution was filtered through a 0.22 µm membrane into a sample vial, and single-strand purification was performed via semi-preparative reversed-phase chromatography. The elution gradient was set from 7% to 30% (acetonitrile: 100 mM TEAA) over 10 min at a flow rate of 5 mL/min. After purification, the eluate was concentrated under vacuum and dried by rotary evaporation at room temperature. Finally, the sample was dissolved in water, and each solution was desalted on a GE Hi-Trap desalting column to elute the final oligonucleotide product. All properties and purity were confirmed using ESI-MS and IEX HPLC, respectively. The concentration was determined via UV measurement with a microplate reader. Equimolar sense strand and antisense strand were combined in a new shipping tube, heated at 95 °C for 5 min, and then slowly annealed to room temperature. The final dsRNA product was obtained after drying by vacuum concentration at room temperature.

The structure of L96 used in the example of the present application is shown in formula I below: 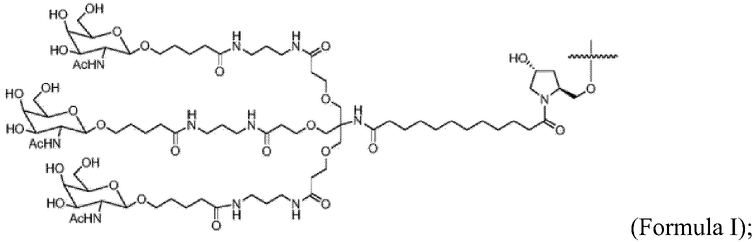 wherein 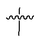 denotes linkage to the 3'-end of the sense strand or antisense strand of the RNA molecule via a phosphodiester bond.

### Example 2: High-throughput screening assay for in vitro activity of FXI-dsRNA

### 2.1 Transfection of HepG2 cells with FXI dsRNA

HepG2 cells were cultured in DMEM medium containing 10% fetal bovine serum in a constant-temperature incubator at 37 °C with 5% CO₂. After cell digestion, the cell density was adjusted to 4×10⁵ cells/ml, and 1 ml of cell suspension was seeded into each well of a 12-well plate. Preparation of 100 ul of transfection complex: 45 µL of Opti-MEM was mixed with 5 µL of dsRNA (naked nucleic acid, unmodified) at various concentrations. 48 µL of Opti-MEM was combined with 2 µL of RNAiMax transfection reagent, and the mixture was allowed to stand for 5 min. The two resultant mixtures were then combined and allowed to stand for 20 min to form the transfection complex. The transfection complex was added to a 12-well plate and further cultured for 24 h in a constant-temperature incubator at 37 °C with 5% CO2. Cells were harvested 24 h after transfection, and RNA was extracted using the TRIZOL method.

### 2.2 Real-time fluorescence quantitative PCR analysis

Cells were lysed 24 h after transfection, and total RNA was extracted using the Vazyme FastPure Cell/Tissue Total RNA Isolation Kit V2 (refer to the Vazyme RC112-01 instruction manual). Reverse transcription into cDNA was performed using Takara PrimeScript RT Master Mix RR036Q. The qPCR primer sequence information is shown in the primer section of the sequence listing at the end of this document. The human GAPDH gene was used as the internal reference gene, and the PCR reaction was performed using a CFX96 fluorescence quantitative PCR instrument from Bio-Rad, USA. In the experiment, normalization was carried out with the Mock group as the control, and the relative expression level of FXI mRNA in the Mock group was set to 1.

### 2.3 Data analysis

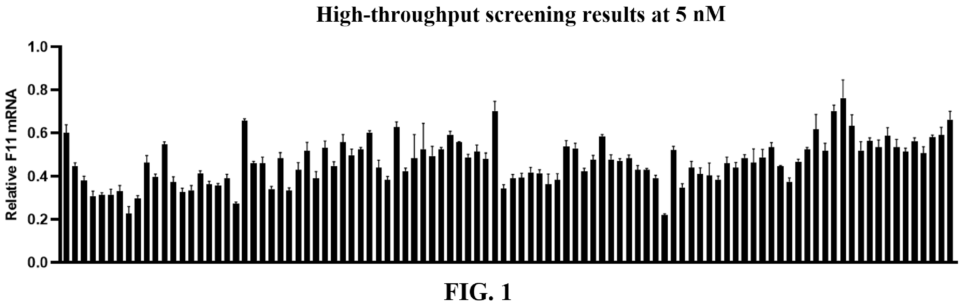

After the PCR reaction was completed, relative quantitative analysis was performed using the reference gene as a standard and CFX96 software, and statistical analysis was performed using GarphPad software. As shown in Table 1 and FIG. 1, these are the results of high-throughput screening validation of dsRNA (naked nucleic acid, unmodified) molecules in HepG2 cells.

**Table 1: High-throughput screening results at a single dose of 5 nM in HepG2 cells**

| No. | Relative FXI activity | ±SEM |
|---|---|---|
| 1 | 0.601 | 0.037 |
| 2 | 0.448 | 0.015 |
| 3 | 0.381 | 0.019 |
| 4 | 0.309 | 0.022 |
| 5 | 0.313 | 0.011 |
| 6 | 0.315 | 0.025 |
| 7 | 0.331 | 0.027 |
| 8 | 0.227 | 0.033 |
| 9 | 0.297 | 0.013 |
| 10 | 0.464 | 0.033 |
| 11 | 0.399 | 0.012 |
| 12 | 0.548 | 0.011 |
| 13 | 0.375 | 0.023 |
| 14 | 0.327 | 0.019 |
| 15 | 0.336 | 0.022 |
| 16 | 0.414 | 0.012 |
| 17 | 0.364 | 0.013 |
| 18 | 0.358 | 0.009 |
| 19 | 0.389 | 0.020 |
| 20 | 0.273 | 0.008 |
| 21 | 0.656 | 0.010 |
| 22 | 0.460 | 0.010 |
| 23 | 0.461 | 0.029 |
| 24 | 0.342 | 0.012 |
| 25 | 0.484 | 0.025 |
| 26 | 0.334 | 0.013 |
| 27 | 0.430 | 0.033 |
| 28 | 0.518 | 0.039 |
| 29 | 0.390 | 0.033 |
| 30 | 0.531 | 0.032 |
| 31 | 0.449 | 0.019 |
| 32 | 0.557 | 0.036 |
| 33 | 0.499 | 0.026 |
| 34 | 0.524 | 0.008 |
| 35 | 0.602 | 0.008 |
| 36 | 0.440 | 0.036 |
| 37 | 0.384 | 0.016 |
| 38 | 0.626 | 0.026 |
| 39 | 0.425 | 0.014 |
| 40 | 0.485 | 0.108 |
| 41 | 0.525 | 0.121 |
| 42 | 0.496 | 0.042 |
| 43 | 0.525 | 0.008 |
| 44 | 0.592 | 0.015 |
| 45 | 0.557 | 0.003 |
| 46 | 0.489 | 0.014 |
| 47 | 0.513 | 0.031 |
| 48 | 0.479 | 0.028 |
| 49 | 0.702 | 0.045 |
| 50 | 0.344 | 0.018 |
| 51 | 0.390 | 0.019 |
| 52 | 0.393 | 0.022 |
| 53 | 0.419 | 0.023 |
| 54 | 0.415 | 0.016 |
| 55 | 0.364 | 0.047 |
| 56 | 0.384 | 0.029 |
| 57 | 0.536 | 0.028 |
| 58 | 0.528 | 0.024 |
| 59 | 0.423 | 0.015 |
| 60 | 0.478 | 0.021 |
| 61 | 0.585 | 0.008 |
| 62 | 0.479 | 0.022 |
| 63 | 0.469 | 0.013 |
| 64 | 0.483 | 0.017 |
| 65 | 0.429 | 0.021 |
| 66 | 0.431 | 0.005 |
| 67 | 0.392 | 0.014 |
| 68 | 0.221 | 0.005 |
| 69 | 0.520 | 0.018 |
| 70 | 0.349 | 0.017 |
| 71 | 0.441 | 0.028 |
| 72 | 0.411 | 0.029 |
| 73 | 0.404 | 0.058 |
| 74 | 0.385 | 0.016 |
| 75 | 0.460 | 0.029 |
| 76 | 0.441 | 0.023 |
| 77 | 0.486 | 0.015 |
| 78 | 0.463 | 0.064 |
| 79 | 0.489 | 0.036 |
| 80 | 0.533 | 0.023 |
| 81 | 0.446 | 0.004 |
| 82 | 0.375 | 0.017 |
| 83 | 0.469 | 0.010 |
| 83 | 0.523 | 0.010 |
| 85 | 0.617 | 0.069 |
| 86 | 0.518 | 0.034 |
| 87 | 0.702 | 0.028 |
| 88 | 0.762 | 0.084 |
| 89 | 0.634 | 0.051 |
| 90 | 0.519 | 0.040 |
| 91 | 0.563 | 0.016 |
| 92 | 0.533 | 0.035 |
| 93 | 0.587 | 0.038 |
| 94 | 0.533 | 0.037 |
| 95 | 0.514 | 0.015 |
| 96 | 0.562 | 0.016 |
| 97 | 0.508 | 0.028 |
| 98 | 0.581 | 0.009 |
| 99 | 0.591 | 0.035 |
| 100 | 0.662 | 0.038 |
| RB2 | 0.512 | 0.016 |
| RB4 | 0.471 | 0.008 |
| RB6 | 0.558 | 0.032 |
| RB7 | 0.481 | 0.061 |
| TJ632 | 0.484 | 0.011 |
| TJ645 | 0.386 | 0.009 |
| TJ772 | 0.485 | 0.017 |

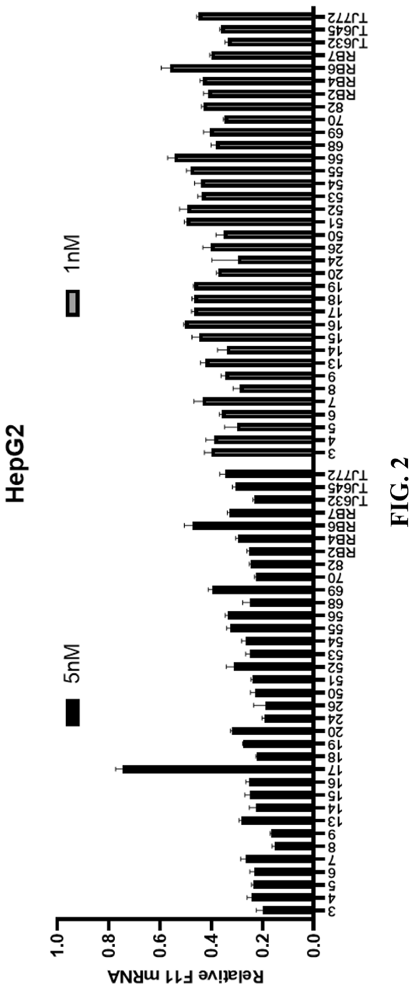

Screening of FXI dsRNA in HepG2 cells revealed 28 preferred sequences, and dsRNA molecules with good efficacy and targeting FXI in both humans and cynomolgus monkeys were retained as candidate sequences. Furthermore, multiple positive control reference sequences were compared with our candidate sequences. Table 2 and FIG. 2 present the single-dose screening results of candidate sequences and positive control sequences in HepG2 cells.

**Table 2: Single-dose screening of unmodified dsRNA in HepG2 cells**

| Sample name | Relative FXI mRNA expression (5nM) | ±SEM | Relative FXI mRNA expression (1nM) | ±SEM |
|---|---|---|---|---|
| 3 | 0.201 | 0.024 | 0.400 | 0.027 |
| 4 | 0.244 | 0.017 | 0.389 | 0.032 |
| 5 | 0.236 | 0.008 | 0.301 | 0.049 |
| 6 | 0.234 | 0.016 | 0.359 | 0.011 |
| 7 | 0.266 | 0.020 | 0.434 | 0.034 |
| 8 | 0.153 | 0.011 | 0.290 | 0.026 |
| 9 | 0.167 | 0.004 | 0.346 | 0.017 |
| 13 | 0.282 | 0.011 | 0.423 | 0.019 |
| 14 | 0.225 | 0.028 | 0.340 | 0.037 |
| 15 | 0.250 | 0.020 | 0.448 | 0.027 |
| 16 | 0.253 | 0.013 | 0.503 | 0.005 |
| 17 | 0.746 | 0.028 | 0.465 | 0.012 |
| 18 | 0.224 | 0.003 | 0.465 | 0.010 |
| 19 | 0.276 | 0.003 | 0.466 | 0.004 |
| 20 | 0.320 | 0.006 | 0.375 | 0.006 |
| 24 | 0.194 | 0.009 | 0.297 | 0.102 |
| 26 | 0.190 | 0.046 | 0.402 | 0.031 |
| 50 | 0.229 | 0.019 | 0.352 | 0.031 |
| 51 | 0.239 | 0.007 | 0.496 | 0.009 |
| 52 | 0.312 | 0.031 | 0.492 | 0.032 |
| 53 | 0.251 | 0.016 | 0.435 | 0.018 |
| 54 | 0.268 | 0.015 | 0.439 | 0.026 |
| 55 | 0.327 | 0.014 | 0.481 | 0.016 |
| 56 | 0.335 | 0.012 | 0.544 | 0.026 |
| 68 | 0.251 | 0.028 | 0.382 | 0.020 |
| 69 | 0.398 | 0.015 | 0.406 | 0.024 |
| 70 | 0.226 | 0.007 | 0.350 | 0.005 |
| 82 | 0.246 | 0.007 | 0.430 | 0.008 |
| RB2 | 0.254 | 0.008 | 0.412 | 0.018 |
| RB4 | 0.297 | 0.009 | 0.433 | 0.011 |
| RB6 | 0.475 | 0.030 | 0.560 | 0.035 |
| RB7 | 0.331 | 0.008 | 0.400 | 0.007 |
| TJ632 | 0.235 | 0.004 | 0.335 | 0.013 |
| TJ645 | 0.307 | 0.012 | 0.363 | 0.005 |
| TJ772 | 0.346 | 0.022 | 0.449 | 0.008 |

The results show that among the 7 candidate positive control sequences, TJ632 exhibited the best activity. Our multiple candidate dsRNA sequences demonstrated superior or equivalent efficacy compared with the positive control TJ632, including dsRNA No. 3, 5, 8, 9, 24, 26, 50, 70 and 82.

### Example 3: Optimization of FXI-dsRNA

### 3.1 Assay for inhibitory activity

To further validate the preferred dsRNA molecules, two distinct modification motifs were applied to the aforementioned preferred sequences. Combinations of fluoro and methoxy modifications were introduced at different positions of the candidate sequences.

The specific modification is selected from any of the following types:
(1) Modification E (dsRNA modified with E is designated as En, where n represents the number of the candidate dsRNA molecule, for instance, dsRNA molecule No. 3 modified with E is denoted as E3):
   sense sequence : nucleotides at positions 7, 9, 10 and 11 starting from the 5' end are 2'-fluoro modified, and nucleotides at all other positions are 2'-O-methyl modified, the first and second phosphoester bonds at the 5' end are phosphorothioate bonds via thiation; for example, taking N to represent any ribonucleotide, the modified sense sequence is shown below: NmsNmsNmNmNmNmNfNmNfNfNfNmNmNmNmNmNmNmNmsNmsNm;
   antisense sequence: nucleotides at positions 2, 6, 14 and 16 starting from the 5' end are 2'-fluoro modified (in other words, nucleotides at positions 2, 6, 14 and 16 starting from the 3' end of the region where the antisense sequence is complementary to the sense strand are 2'-fluoro modified), nucleotides at all other positions are 2'-O-methyl modified, and the first and second phosphoester bonds at both the 5' end and the 3' end are converted into phosphorothioate bonds via thiation; for example, taking N to represent any ribonucleotide, the modified antisense sequence is shown below: NmsNfsNmNmNmNfNmNmNmNmNmNmNmNfNmNfNmNmNmNmNmsNmsNm.
(2) Modification E-1 (dsRNA modified with E-1 is designated as En-1, where n represents the number of the candidate dsRNA molecule, for instance, dsRNA molecule No. 3 modified with E-1 is denoted as E3-1),
   the only difference between modification E-1 and modification E1 is that the 6th nucleotide starting from the 5' end of the antisense strand of dsRNA with modification E-1 is a 2'-O-methyl modified nucleotide instead of a 2'-fluoro modified nucleotide, as exemplified below:
   modified sense sequence: NmsNmsNmNmNmNmNfNmNfNfNfNmNmNmNmNmNmNmNmsNmsNm;
   modified antisense sequence: NmsNfsNmNmNmNmNmNmNmNmNmNmNmNfNmNfNmNmNmNmNmsNmsNm.

In the sequence modifications described herein: s stands for phosphorothioate bond, m stands for 2'-O-methyl modification, and f stands for 2'-fluoro modification.

In addition, the antisense compound ISIS-416858 adopts the structure and modification pattern disclosed in CN109797150A, namely a "5-10-5" structure. Each flanking segment comprises five 2'-O-methoxyethyl modified nucleotides, the middle 10 deoxyribonucleotides and fully phosphorothioated, methylcytosine replacing cytosine.

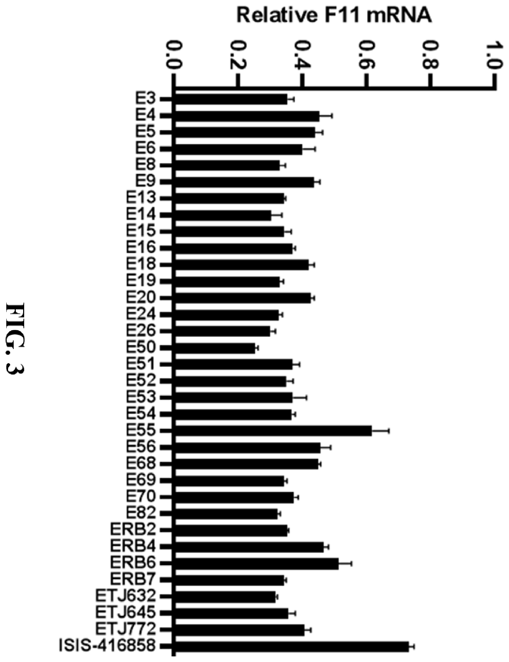

HepG2 cells were used for transfection. The procedures for synthesis, transfection and quantitative PCR detection were consistent with those described in Example 2. Table 3 and FIG. 3 present the mean expression levels of the target gene relative to the blank control group (the relative mRNA expression level of the blank control group was set to 1).

**Table 3: Single-dose (1 nM) screening of modified dsRNA in HepG2 cells**

| Sample name | Relative FXI mRNA expression | ±SEM |
|---|---|---|
| E3 | 0.355 | 0.020 |
| E4 | 0.454 | 0.039 |
| E5 | 0.439 | 0.025 |
| E6 | 0.401 | 0.039 |
| E8 | 0.329 | 0.019 |
| E9 | 0.437 | 0.018 |
| E13 | 0.343 | 0.006 |
| E14 | 0.302 | 0.035 |
| E15 | 0.345 | 0.021 |
| E16 | 0.371 | 0.008 |
| E18 | 0.420 | 0.018 |
| E19 | 0.329 | 0.013 |
| E20 | 0.427 | 0.011 |
| E24 | 0.326 | 0.013 |
| E26 | 0.301 | 0.016 |
| E50 | 0.252 | 0.011 |
| E51 | 0.369 | 0.023 |
| E52 | 0.351 | 0.021 |
| E53 | 0.371 | 0.043 |
| E54 | 0.368 | 0.011 |
| E55 | 0.618 | 0.053 |
| E56 | 0.455 | 0.034 |
| E68 | 0.450 | 0.008 |
| E69 | 0.344 | 0.009 |
| E70 | 0.375 | 0.013 |
| E82 | 0.323 | 0.009 |
| ERB2 | 0.353 | 0.005 |
| ERB4 | 0.467 | 0.015 |
| ERB6 | 0.514 | 0.040 |
| ERB7 | 0.342 | 0.008 |
| ETJ632 | 0.318 | 0.005 |
| ETJ645 | 0.358 | 0.021 |
| ETJ772 | 0.405 | 0.022 |
| ISIS-416858 | 0.734 | 0.016 |

It can be seen that compared with the chemically modified positive control sequences, multiple candidate sequences including E8, E14, E19, E24, E26 and E50 exhibited equivalent or better activity than the optimal positive control ETJ632. This result further demonstrates that the selected preferred sequences have the potential to effectively downregulate the nucleic acid level of the FXI gene.

### Example 4: In vitro efficacy assay of GalNAc-dsRNA

Candidate modified dsRNAs were conjugated with GalNAc to form GAL-dsRNA complexes, i.e., each dsRNA was subjected to modification G.

Modification G refers to the conjugation of L96 to the 3' end of the dsRNA sense sequence, including modification GE and modification GE-1, wherein:
modification GE is shown below, where "-L96" indicates that L96 is conjugated to the 3' end of the dsRNA sense sequence:
modified sense sequence: NmsNmsNmNmNmNmNfNmNfNfNfNmNmNmNmNmNmNmNmNmNm-L96;
modified antisense sequence: NmsNfsNmNmNmNfNmNmNmNmNmNmNmNfNmNfNmNmNmNmNmsNmsNm;
dsRNA with modification GE is designated as GEn, where n represents the number of the candidate dsRNA molecule, for example, dsRNA No. 3 molecule modified with GE is denoted as GE3;

GE-1 is as shown below:
modified sense sequence: NmsNmsNmNmNmNmNfNmNfNfNfNmNmNmNmNmNmNmNmNmNm-L96;
modified antisense sequence: NmsNfsNmNmNmNmNmNmNmNmNmNmNmNfNmNfNmNmNmNmNmsNmsNm;

Similarly, "-L96" means L96 is conjugated to the 3' end of the dsRNA sense sequence via a phosphoester bond, dsRNA with modification GE-1 is designated as GEn-1, where n represents the number of the candidate dsRNA molecule, for instance, dsRNA molecule No. 3 modified with GE-1 is denoted as GE3-1.

We further validated the activity of the G-modified candidate complex in HepG2 cells.

### 4.1 Detection of mRNA expression levels in HepG2 cells

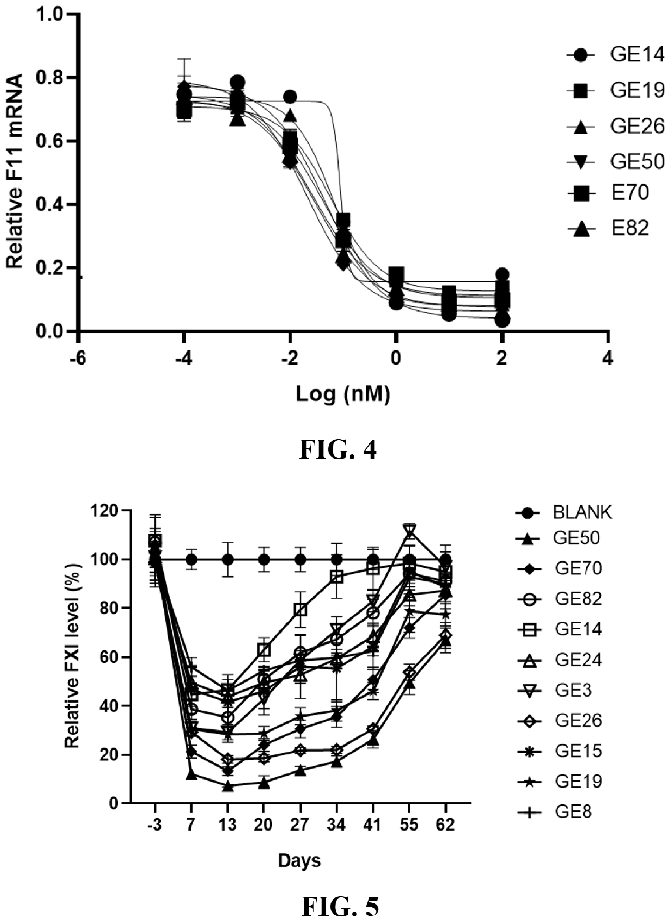

The detection steps are the same as in Example 2, with the human GAPDH gene as the internal reference gene, and the detection primers used are shown in the primer section of the sequence listing at the end of this document. Table 4 shows the average mRNA levels in HepG2 cells treated with G-modified dsRNA relative to the target gene expression levels in the untreated group (with the relative mRNA expression level in the untreated group set to 1). The statistical results are shown in FIG. 4.

**Table 4 IC50 data of some GalNAc-conjugated dsRNAs in HepG2 cells**

| Sample name | IC₅₀ (nM) |
|---|---|
| GE14 | 0.089 |
| GE19 | 0.059 |
| GE26 | 0.066 |
| GE50 | 0.026 |
| GE70 | 0.040 |
| GE82 | 0.030 |

It can be seen that the IC50 values of all GalNAc-conjugated dsRNAs in HepG2 cells were below 0.1 nM, which further verifies that the candidate sequences exert potent inhibitory activity against the target gene.

### Example 5: In vivo efficacy evaluation

SPF-grade male homozygous humanized FXI mice aged 6 to 8 weeks (purchased from Shanghai Model Organisms Center, Inc.) were used in this study. The mice were randomly divided into groups with 6 animals per group. A single subcutaneous injection was administered to each group. Mice in different groups received distinct GE-modified dsRNAs, while the Blank group was injected with normal saline. Blood samples were collected on Day 7 and Day 13 after administration. Serum was obtained via retro-orbital bleeding for the detection of FXI protein levels.

The specific dosages are shown in Table 5.

**Table 5 Single-dose regimen at 3 mpk**

| Drug name | Dosage | Humanized FXI mice |
|---|---|---|
| Blank | Normal saline | |
| GE3 | | |
| GE8 | | |
| GE14 | | |
| GE19 | | N=6, male |
| GE24 | 3mpk | |
| GE26 | | |
| GE50 | | |
| GE70 | | |
| GE82 | | |

### 5.1 ELISA Detection of FXI Protein

Serum samples were diluted at a ratio of 1:500, and the residual FXI content in plasma of each group was measured using the ab108834 ELISA kit (Abcam). All operations were performed in accordance with the product manual of ab108834 ELISA kit. The absorbance was read at 450 nm using a microplate reader, and data analysis was conducted with GraphPad software. The percentage inhibition was calculated relative to the untreated group. The results are presented in Table 6. FIG. 5 shows the relative expression levels of FXI protein in plasma of each group after normalization against the blank control group.

**Table 6: Inhibition rate of FXI protein in plasma after a single 3mpk dose**

| Drug name | Day 7 | Day 13 |
|---|---|---|
| | Inhibition rate (%) | Inhibition rate (%) |
| GE3 | 53.9 | 52.3 |
| GE8 | 44.0 | 53.7 |
| GE15 | 53.1 | 58.3 |
| GE14 | 55.3 | 53.4 |
| GE19 | 69.4 | 71.6 |
| GE24 | 50.5 | 56.2 |
| GE26 | 70.7 | 81.8 |
| GE50 | 87.9 | 92.8 |
| GE70 | 78.6 | 86.7 |
| GE82 | 61.2 | 64.7 |

It is evident that, after a single administration at 3 mpk, the plasma FXI levels in mice of the groups treated with the candidate dsRNA molecules were significantly lower than those in the blank control group. On day 13, the inhibition rates of GE26, GE50 and GE70 were all above 80%, with GE50 showing the highest inhibition rate of 92.8%. Meanwhile, on day 13, the inhibition rate was >50% in all treatment groups. These results demonstrate that our multiple dsRNA molecules designed against FXI can effectively inhibit the expression and secretion of FXI protein in the liver, and represent a potential therapeutic drug targeting the FXI gene.

### Example 6: In vivo efficacy evaluation of different ligands

We further validated the effects of different ligand conjugates using candidate dsRNAs. Table 7 shows the dsRNA sequence information. The dsRNAs were modified with either LN-E05 or GN-E05. Modification LN-E05 is identical to modification GE-1 described in Example 4. The details of modification GN-E05 are as follows:
modified sense sequence: NmsNmsNmNmNmNmNfNmNfNfNfNmNmNmNmNmNmNmNmNmNms-ligand 1;
modified antisense sequence: NmsNfsNmNmNmNmNmNmNmNmNmNmNmNfNmNfNmNmNmNmNmsNmsNm;

That is, the only difference between modification GN-E05 and modification LN-E05 is that the L96 ligand conjugated to the 3' end of the sense strand was replaced with ligand 1, and ligand 1 was linked to the 3' end of the sense strand via a phosphorothioate bond.

**Table 7 Synthesized oligonucleotides**

| dsRNA sequence | Base sequence | Modification motif | Ligand structure |
|---|---|---|---|
| LN50-E05 | Sense strand: SEQ ID NO: 58 | LN-E05 | L96 |
| GN50-E05 | | GN-E05 | Ligand 1 |
| | Antisense strand: SEQ ID NO: 177 | | |
| LN26-E05 | Sense strand: SEQ ID NO: 34 | LN-E05 | L96 |
| GN26-E05 | | GN-E05 | Ligand 1 |
| | Antisense strand: SEQ ID NO: 153 | | |

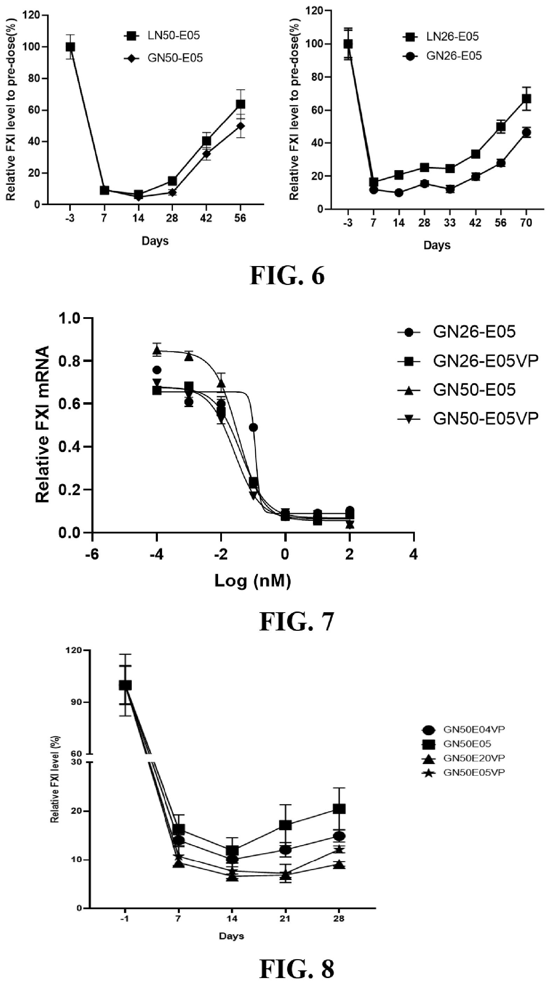

Grouping was performed in accordance with Table 7. All groups received a single subcutaneous injection at a dose of 3 mpk. Blood samples were collected at different time points after administration, and the plasma was used for the detection of FXI protein levels. The percentage residual rate was calculated relative to the pre-administration baseline. The results are shown in FIG. 6. After single administration at 3 mpk, the dsRNA conjugated with ligand 1 exhibited a significantly higher inhibition rate against FXI protein than the dsRNA conjugated with L96 (paired t-test, p<0.05).

The structure of ligand 1 is shown in Formula II: 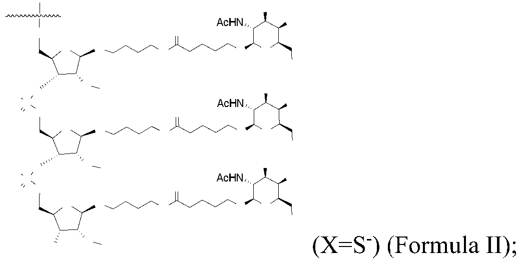 wherein the wavy line indicates the linkage to the 3' end of the dsRNA sense strand via a phosphorothioate bond. The molecule of formula II is a trivalent GalNAc conjugate synthesized from two equivalents of compound 6 and one equivalent of compound 8 using conventional raw materials and synthetic methods known in the art. The synthetic procedures for compound 6 and compound 8 are described below.

### 1. Synthesis of compound 6

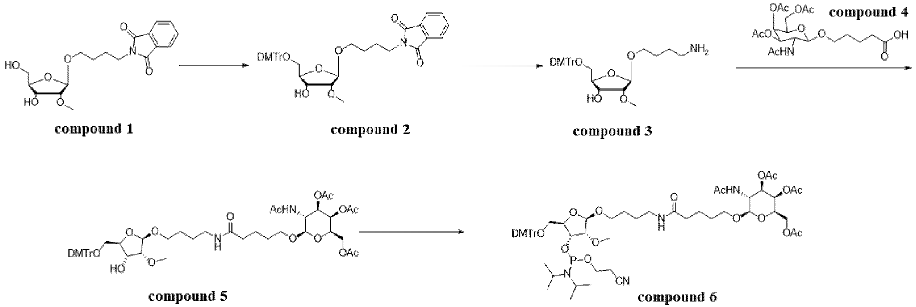

### (1) Synthesis of compound 2:

To a three-necked flask were added 1.2 g of compound 1 and 12 mL of pyridine. After stirring to achieve dissolution, 1.2 g of DMTrCl was added. The mixture was stirred at room temperature for 1 h under nitrogen protection. Methanol (0.6 mL) was slowly added to the reaction mixture to quench excess DMTrCl. After stirring at room temperature for 15 min, 303.5 mg of NaHCO3 was supplemented. The mixture was concentrated to afford a crude product. DCM/H2O were added, followed by stirring for dissolution. The mixture was washed with water, and the organic phase was dried over anhydrous sodium sulfate and then filtered. The collected organic phase was concentrated under vacuum to give crude compound 2, which was used directly in the next reaction step.

### (2) Synthesis of compound 3:

Ethanol (24 mL) was added to the crude compound 2. The mixture was gradually heated to 50 °C and stirred until fully dissolved. Hydrazine hydrate (0.75 mL) was then added, and the reaction was stirred overnight at 50 °C. Afterwards, the system was cooled to room temperature and stirred for another half an hour, during which a large amount of white solid gradually precipitated. The mixture was filtered, and the filter cake was washed with ethanol. The resulting filtrate was concentrated under reduced pressure, redissolved in DCM/H2O, and then separated. The organic phase was collected, washed with saturated brine, dried over anhydrous sodium sulfate and filtered. The collected organic phase was concentrated under vacuum to yield crude compound 3, which was carried forward to the subsequent reaction directly.

### (3) Synthesis of compound 5:

To the crude compound 3 were sequentially added 0.9 g of compound 4, DCM (24 mL), 0.84 mL of Et3N and 2.3 g of HBTU. The reaction mixture was stirred at room temperature for 2 h under nitrogen protection. The reaction was quenched with 9 mL of water. The organic phase was separated, washed with saturated brine, dried over anhydrous sodium sulfate and filtered. The collected organic phase was concentrated under vacuum, and the residue was purified by reversed-phase preparative chromatography (75%ACN-H2O) to give 1.4 g of compound 5. The overall yield over three steps was 44.2%. ESI-MS: m/z 1025.6 [M⁺OAc⁻]⁻.

### (4) Synthesis of compound 6:

1.4 g of compound 5 was dissolved in DCM (14 mL) and cooled to 0±2 °C. 524.7 mg of 2-cyanoethyl-N,N,N',N'-tetraisopropylphosphorodiamidite was added, and then 81.3 mg of 1H-tetrazole was added. The reaction mixture was first stirred at 0 ± 2 °C for 15 min, then warmed to room temperature and stirred for an additional 2 h. The reaction mixture was cooled to 0 ± 2 °C and quenched with 14 mL of 5% NaHCO3. The organic phase was separated, washed with saturated brine (1x14mL) at 0 ± 2 °C, dried over anhydrous sodium sulfate and filtered. The collected organic phase was concentrated under vacuum. The resulting crude product was redissolved in DCM/MTBE, and the solution was slowly added dropwise into vigorously stirred n-heptane (hept.). A large amount of viscous oily substance gradually deposited on the wall and bottom of the flask. After standing for 10 min, the supernatant was decanted. The crude product was purified by column chromatography using EA:hept.=10:1-2:1 as eluent. After concentration and drying, 560 mg of white solid powder was obtained with a yield of 32% and a purity of 98.19% determined by phosphorus NMR. ESI-MS: m/z 1225.4 [M⁺OAc⁻]⁻.

### 2. Synthesis of compound 8

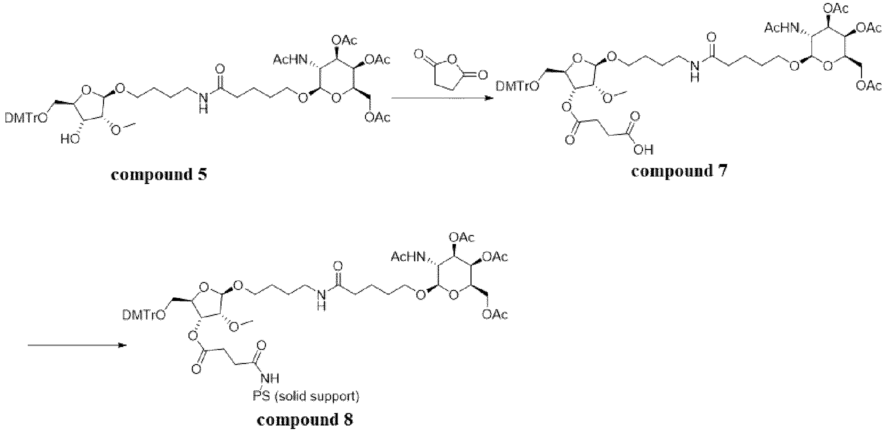

### (1) Synthesis of compound 7:

2 g of compound 5 was dissolved in DCM (20 mL), and 248.4 mg of succinic anhydride and 0.69 mL of Et3N were added sequentially. Finally, 25.3 mg of DMAP was added. The mixture was stirred at room temperature for 16 h. HPLC monitoring indicated that a large amount of starting material remained, so additional 310.4 mg of succinic anhydride and 0.69 mL of Et3N were supplemented. Finally, 25.3 mg of DMAP was added and the mixture was further stirred at room temperature for 36 h. The reaction mixture was cooled to 0 ± 2 °C, then ice water and DCM were added successively, followed by 40 mL of 1% aqueous HOAc solution. After stirring for 10 min, the organic phase was separated. It was washed sequentially with 1% aqueous HOAc solution and water, dried over anhydrous sodium sulfate and filtered. The collected organic phase was concentrated under vacuum to give a crude product. The crude product was purified by column chromatography using DCM:MeOH=70:1-20:1 as eluent. After concentration and drying, 0.9 g of white solid powder was obtained with a yield of 40.7%. No residual succinic acid was detected, and the residual content of succinic anhydride was 0.26%. ESI-MS: m/z 1065.7[M⁻H]⁻.

### (2) Synthesis of compound 8:

To a three-necked flask were added 300 mg of compound 7, 72.7 mg of DIPEA, 106 mg of HBTU and 10 mL of acetonitrile. The mixture was stirred at 25 °C for 10 min, then 650 mg of solid support PS was added, and stirring was continued at 25 °C for 24 h. Upon completion of the reaction, the mixture was filtered. The filter cake was rinsed with acetonitrile and concentrated under vacuum to remove the solvent, affording 850 mg of solid. A 700 mg portion of the solid was transferred to a three-necked flask, and 1.74 g of acetic anhydride, 4.15 mg of DMAP, 103 mg of triethylamine and 10 mL of pyridine were added. The mixture was stirred at 25 °C for 4 h. Upon completion of the reaction, the mixture was filtered. The filter cake was washed successively with acetonitrile, methanol and acetonitrile, then the filter cake was collected and concentrated under vacuum to remove the solvent, yielding 750 mg of compound 8. The measured loading capacity was 254.82 µmol/g.

### 3. Preparation of oligonucleotide sequence conjugated with ligand 1

Compound 8 reacted sequentially with 2 equivalents of compound 6, or with 3 equivalents of compound 6 in sequence. Conventional solid-phase synthesis cycles (deprotection, coupling, oxidation and capping) were performed to give the precursor (or intermediate) of ligand 1 immobilized on the solid support. Subsequent solid-phase synthesis cycles were carried out using the corresponding 2'-modified monomers. After the synthesis was completed, the entire synthesized molecule was dissociated from the solid support via ammonolysis, to give the oligonucleotide sequence conjugated with ligand 1.

### Example 7: In vitro and in vivo efficacy evaluation of different modification motifs

We further evaluated the effects of different modification motifs using dsRNAs sharing the base sequences of Duplex No. 50 and Duplex No. 26. The specific molecules used are shown in Table 8. It can be seen that all molecules in Table 8 were conjugated with Ligand 1 at the 3' end of the sense strand via a phosphorothioate bond, and the nucleotide at the 5' end of the antisense strand was modified with 5'-vp.

**Table 8: Synthesized oligonucleotides**

| dsRNA No. | Base sequence | Chemical modification |
|---|---|---|
| GN50-E20VP | Sense strand: SEQ ID NO: 58 | GN-E20VP |
| GN50-E04VP | | GN-E04VP |
| GN50-E05VP | Antisense strand: SEQ ID NO: 177 | GN-E05VP |
| GN50-E05 | | GN-E05 |
| GN26-E20VP | Sense strand: SEQ ID NO: 34 | GN-E20VP |
| GN26-E04VP | | GN-E04VP |
| | Antisense strand: SEQ ID NO: 153 | |
| GN26-E05VP | | GN-E05VP |
| GN26-E05 | | GN-E05 |

The modification motifs in Table 8 are shown below:
GN-E20VP:
   sense sequence: NmsNmsNmNmNmNmNfNmNfNfNfNfNmNmNmNmNmNmNmNmNms-ligand 1
   antisense sequence: vp-NmsNfsNmNmNmNmNmNmNmNmNmNmNmNfNmNfNmNmNmNmNmsNmsNm;
GN-E04VP:
   sense sequence: NmsNmsNmNmNmNmNfNmNfNfNfNmNmNmNmNmNmNmNmNmNms-ligand 1
   antisense sequence: vp-NmsNfsNmNmNmNfNmNmNmNmNmNmNmNfNmNfNmNmNmNmNmsNmsNm;
GN-E05VP:
   sense sequence: NmsNmsNmNmNmNmNfNmNfNfNfNmNmNmNmNmNmNmNmNmNms-ligand 1
   antisense sequence: vp-NmsNfsNmNmNmNmNmNmNmNmNmNmNmNfNmNfNmNmNmNmNmsNmsNm.

Modification GN-E05 is the same as in Example 6, except that the only difference from modification GN-E05VP is that the 5' end of the antisense strand of GN-E05 has no vinyl phosphate modification.

### 7.1 In vitro efficacy evaluation of different modification motifs

First, the effects of various modifications listed in Table 8 on efficacy were evaluated in HepG2 cells in vitro. The experimental methods were performed in accordance with Example 4. Table 4 presents the average mRNA relative expression levels of the target gene in each treatment group relative to the untreated group (with the relative mRNA expression level of the untreated group set as 1). The in vitro assays were performed as independent replicate experiments in different batches, and the statistical results are shown in Table 9 and Table 10.

**Table 9 IC50 data of dsRNA with modification 5VP in HepG2 cells**

| Sample name | IC₅₀ (nM) |
|---|---|
| GN26-E05 | 0.117 |
| GN26-E05VP | 0.041 |
| GE50-E05 | 0.034 |
| GN50-E05VP | 0.026 |

**Table 10 IC50 data of dsRNA with different motif modifications in HepG2 cells**

| Sample name | IC₅₀ (nM) |
|---|---|
| GN26-E04VP | 0.167 |
| GN26-E05VP | 0.099 |
| GN26-E20VP | 0.044 |

The in vitro results are shown in Table 9 and FIG. 7, indicating that modification 5-VP helps to further improve the efficacy. Meanwhile, with the same ligand and base sequence, the modification motifs of GN-E05VP and GN-E20VP were significantly superior to those of GN-E04VP (as shown in Table 10). Furthermore, the modification motif of GN-E20VP was also significantly better than that of GN-E05VP.

### 7.2 In vivo efficacy evaluation of different modification motifs

The experimental protocol was referred to Example 5. Groups specified in Table 8 received a single subcutaneous injection of No. 50 dsRNA with different modifications at a dose of 1 mpk. Blood samples were collected on Day 7, 14, 21 and 28 after administration, and plasma was used for the detection of FXI protein levels. The percentage residual rate was calculated relative to the pre-administration baseline. The results are presented in Table 11 and FIG. 8.

**Table 11: Residual rate of plasma FXI protein after single administration at 1 mpk**

| Drug name | Day 7 | Day 14 | Day 28 |
|---|---|---|---|
| | Residual rate (%) | Residual rate (%) | Residual rate (%) |
| GN50-E05 | 16.3 | 11.9 | 20.4 |
| GN50-E04VP | 14.0 | 10.1 | 14.9 |
| GN50-E05VP | 10.7 | 7.7 | 12.2 |
| GN50-E20VP | 9.4 | 6.6 | 9.1 |

As indicated by the results of the modified molecule No. 50, following single administration at 1 mpk, with the same dsRNA base sequence, the modification motifs E05VP and E20VP were superior to E04VP (P<0.05), and E20VP was further superior to E05VP (P < 0.05). Meanwhile, the results show that modification 5'-VP helps to further improve the efficacy.

Furthermore, we compared the efficacy of different modification motifs of molecule No. 26 in vivo (as shown in Table 8). Blood samples were collected on days 7 and 14 after administration, and plasma was used for the detection of FXI protein levels. The percentage residual rate was calculated relative to the pre-administration baseline. The results are shown in Table 12.

**Table 12: Residual rate of plasma FXI protein after single administration at 1 mpk**

| Drug name | Day 7 | Day 14 |
|---|---|---|
| | Residual rate (%) | Residual rate (%) |
| GN26-E04VP | 20.6 | 24.7 |
| GN26-E05VP | 17.2 | 18.0 |
| GN26-E20VP | 12.4 | 14.0 |

The superior efficacy of E05VP and E20VP over E04VP was further confirmed with molecule No. 26, and the E20VP motif exhibited the best efficacy overall. In addition, modification 5'-VP contributed to further improved efficacy.

The sequences used in the above examples of the present application are listed in the following Sequence Listing. It should be understood that the following sequences are merely exemplary sequences for the embodiments of this application and shall not be construed as any limitation thereto. The nucleic acid sequences in the following Sequence Listing can represent DNA or RNA sequences. When they represent RNA sequences, "T" represents uridine. Furthermore, in the context of RNA, unless otherwise specified, both "T" and "U" refer to uracil or uridine.

### Sequence Listing:

| dsRNA No. | Sense sequence (5'->3') | Sense sequenc e ID | Antisense sequence (5'->3') | Antisense sequence ID |
|---|---|---|---|---|
| 1 | CCGATGGTTTACTTGTG TCCT | SEQ ID NO: 1 | AGGACACAAGTAAACCAT CGGGT | SEQ ID NO: 120 |
| 2 | CTTTCAAGCAATGCTCA CACC | SEQ ID NO: 2 | GGTGTGAGCATTGCTTGA AAGAA | SEQ ID NO: 121 |
| 3 | GCAATGCTCACACCAA ATAAG | SEQ ID NO: 3 | CTTATTTGGTGTGAGCATT GCTT | SEQ ID NO: 122 |
| 3' | GCAATGCTCACACCAA ATAAT | SEQ ID NO: 4 | ATTATTTGGTGTGAGCATT GCTT | SEQ ID NO: 123 |
| 4 | CAATGCTCACACCAAA TAAGC | SEQ ID NO: 5 | GCTTATTTGGTGTGAGCAT TGCT | SEQ ID NO: 124 |
| 4' | CAATGCTCACACCAAA TAAGT | SEQ ID NO: 6 | ACTTATTTGGTGTGAGCAT TGCT | SEQ ID NO: 125 |
| 5 | ACCAAATAAGCGCTTG CAACA | SEQ ID NO: 7 | TGTTGCAAGCGCTTATTTG GTGT | SEQ ID NO: 126 |
| 6 | CCAAATAAGCGCTTGC AACAA | SEQ ID NO: 8 | TTGTTGCAAGCGCTTATTT GGTG | SEQ ID NO: 127 |
| 7 | CAAAGACATTTATGTG GACCT | SEQ ID NO: 9 | AGGTCCACATAAATGTCTT TGTT | SEQ ID NO: 128 |
| 8 | AGGGCATAAACTATAA CAGCT | SEQ ID NO: 10 | AGCTGTTATAGTTTATGCC CTTC | SEQ ID NO: 129 |
| 9 | GGGCATAAACTATAAC AGCTC | SEQ ID NO: 11 | GAGCTGTTATAGTTTATGC CCTT | SEQ ID NO: 130 |
| 9' | GGGCATAAACTATAAC AGCTT | SEQ ID NO: 12 | AAGCTGTTATAGTTTATGC CCTT | SEQ ID NO: 131 |
| 10 | ATGCCAAGAAAGATGC ACGGA | SEQ ID NO: 13 | TCCGTGCATCTTTCTTGGC ATTC | SEQ ID NO: 132 |
| 11 | ACTTTTTCACGTACGCC ACAA | SEQ ID NO: 14 | TTGTGGCGTACGTGAAAA AGTGG | SEQ ID NO: 133 |
| 12 | ATTTCCTACACAAGCTC ATTC | SEQ ID NO: 15 | GAATGAGCTTGTGTAGGA AATGG | SEQ ID NO: 134 |
| 13 | TGCTCACACCAAATAA GCGCT | SEQ ID NO: 16 | AGCGCTTATTTGGTGTGA GCATT | SEQ ID NO: 135 |
| 14 | AAATAAGCGCTTGCAA CAAAG | SEQ ID NO: 17 | CTTTGTTGCAAGCGCTTAT TTGG | SEQ ID NO: 136 |
| 14' | AAATAAGCGCTTGCAA CAAAT | SEQ ID NO: 18 | ATTTGTTGCAAGCGCTTAT TTGG | SEQ ID NO: 137 |
| 15 | CATGAAGGGCATAAAC TATAA | SEQ ID NO: 19 | TTATAGTTTATGCCCTTCA TGTC | SEQ ID NO: 138 |
| 16 | GAAGGGCATAAACTAT AACAG | SEQ ID NO: 20 | CTGTTATAGTTTATGCCCT TCAT | SEQ ID NO: 139 |
| 16' | GAAGGGCATAAACTAT AACAT | SEQ ID NO: 21 | ATGTTATAGTTTATGCCCT TCAT | SEQ ID NO: 140 |
| 17 | AAGGGCATAAACTATA ACAGC | SEQ ID NO: 22 | GCTGTTATAGTTTATGCCC TTCA | SEQ ID NO: 141 |
| 18 | GGCATAAACTATAACA GCTCA | SEQ ID NO: 23 | TGAGCTGTTATAGTTTATG CCCT | SEQ ID NO: 142 |
| 19 | AAGAAAGATGCACGGA TGACG | SEQ ID NO: 24 | CGTCATCCGTGCATCTTTC TTGG | SEQ ID NO: 143 |
| 20 | CTGCCACTTTTTCACGT ACGC | SEQ ID NO: 25 | GCGTACGTGAAAAAGTGG CAGTG | SEQ ID NO: 144 |
| 20' | CTGCCACTTTTTCACGT ACGT | SEQ ID NO: 26 | ACGTACGTGAAAAAGTGG CAGTG | SEQ ID NO: 145 |
| 21 | AGCACCCTTATTAAGA ATTGC | SEQ ID NO: 27 | GCAATTCTTAATAAGGGT GCTTT | SEQ ID NO: 146 |
| 22 | GTTTCTGGTGAATGTGT GACT | SEQ ID NO: 28 | AGTCACACATTCACCAGA AACTG | SEQ ID NO: 147 |
| 23 | GGAGGGGACATTACTA CGGTC | SEQ ID NO: 29 | GACCGTAGTAATGTCCCC TCCTT | SEQ ID NO: 148 |
| 24 | ACTGCCAAGAGTGAAT AGGAC | SEQ ID NO: 30 | GTCCTATTCACTCTTGGCA GTGT | SEQ ID NO: 149 |
| 24' | ACTGCCAAGAGTGAAT AGGAT | SEQ ID NO: 31 | ATCCTATTCACTCTTGGCA GTGT | SEQ ID NO: 150 |
| 25 | TTTCAAGCAATGCTCAC ACCA | SEQ ID NO: 32 | TGGTGTGAGCATTGCTTG AAAGA | SEQ ID NO: 151 |
| 26 | TCACACCAAATAAGCG CTTGC | SEQ ID NO: 33 | GCAAGCGCTTATTTGGTGT GAGC | SEQ ID NO: 152 |
| 26' | TCACACCAAATAAGCG CTTGT | SEQ ID NO: 34 | ACAAGCGCTTATTTGGTGT GAGC | SEQ ID NO: 153 |
| 27 | ACATGAAGGGCATAAA CTATA | SEQ ID NO: 35 | TATAGTTTATGCCCTTCAT GTCT | SEQ ID NO: 154 |
| 28 | TGAAGGGCATAAACTA TAACA | SEQ ID NO: 36 | TGTTATAGTTTATGCCCTT CATG | SEQ ID NO: 155 |
| 29 | GTTGCCAAGAGTGCTC AAGAA | SEQ ID NO: 37 | TTCTTGAGCACTCTTGGCA ACTG | SEQ ID NO: 156 |
| 30 | GCACCCTTATTAAGAAT TGCA | SEQ ID NO: 38 | TGCAATTCTTAATAAGGGT GCTT | SEQ ID NO: 157 |
| 31 | ACAAGGTCTTTTCAGG ATGAT | SEQ ID NO: 39 | ATCATCCTGAAAAGACCTT GTTG | SEQ ID NO: 158 |
| 32 | ACACAAGCTCATTCAG AGGAG | SEQ ID NO: 40 | CTCCTCTGAATGAGCTTGT GTAG | SEQ ID NO: 159 |
| 33 | GTCCACTGCCACTTTTT CACG | SEQ ID NO: 41 | CGTGAAAAAGTGGCAGTG GACGT | SEQ ID NO: 160 |
| 34 | GAAAAGCACCCTTATT AAGAA | SEQ ID NO: 42 | TTCTTAATAAGGGTGCTTT TCTT | SEQ ID NO: 161 |
| 35 | AAAAGCACCCTTATTA AGAAT | SEQ ID NO: 43 | ATTCTTAATAAGGGTGCTT TTCT | SEQ ID NO: 162 |
| 36 | TCCACTGCCACTTTTTC ACGT | SEQ ID NO: 44 | ACGTGAAAAAGTGGCAGT GGACG | SEQ ID NO: 163 |
| 37 | CACTGCCACTTTTTCAC GTAC | SEQ ID NO: 45 | GTACGTGAAAAAGTGGCA GTGGA | SEQ ID NO: 164 |
| 38 | TTTCCTACACAAGCTCA TTCA | SEQ ID NO: 46 | TGAATGAGCTTGTGTAGG AAATG | SEQ ID NO: 165 |
| 39 | CCACTGCCACTTTTTCA CGTA | SEQ ID NO: 47 | TACGTGAAAAAGTGGCAG TGGAC | SEQ ID NO: 166 |
| 40 | GATGCACGGATGACGT CCACT | SEQ ID NO: 48 | AGTGGACGTCATCCGTGC ATCTT | SEQ ID NO: 167 |
| 41 | CTGAGAGTGGATTGCC CAGTA | SEQ ID NO: 49 | TACTGGGCAATCCACTCTC AGAT | SEQ ID NO: 168 |
| 42 | AGAAAGATGCACGGAT GACGT | SEQ ID NO: 50 | ACGTCATCCGTGCATCTTT CTTG | SEQ ID NO: 169 |
| 43 | GAAAGATGCACGGATG ACGTC | SEQ ID NO: 51 | GACGTCATCCGTGCATCTT TCTT | SEQ ID NO: 170 |
| 44 | AGATGCACGGATGACG TCCAC | SEQ ID NO: 52 | GTGGACGTCATCCGTGCA TCTTT | SEQ ID NO: 171 |
| 45 | GAAACTGTGCACCAAT GCCGT | SEQ ID NO: 53 | ACGGCATTGGTGCACAGT TTCTG | SEQ ID NO: 172 |
| 46 | TCCGCTGCCAGTTTTTT ACCT | SEQ ID NO: 54 | AGGTAAAAAACTGGCAGC GGACG | SEQ ID NO: 173 |
| 47 | AGTTTTTTACCTATACC CCAG | SEQ ID NO: 55 | CTGGGGTATAGGTAAAAA ACTGG | SEQ ID NO: 174 |
| 48 | GTTTTTTACCTATACCC CAGC | SEQ ID NO: 56 | GCTGGGGTATAGGTAAAA AACTG | SEQ ID NO: 175 |
| 49 | TTTTTTACCTATACCCC AGCC | SEQ ID NO: 57 | GGCTGGGGTATAGGTAAA AAACT | SEQ ID NO: 176 |
| 50 | AAGTGTTACTTAAAGCT TTCT | SEQ ID NO: 58 | AGAAAGCTTTAAGTAACA CTTGC | SEQ ID NO: 177 |
| 51 | AGCTTTCTTCAAACGGA TCTC | SEQ ID NO: 59 | GAGATCCGTTTGAAGAAA GCTTT | SEQ ID NO: 178 |
| 51' | AGCTTTCTTCAAACGGA TCTT | SEQ ID NO: 60 | AAGATCCGTTTGAAGAAA GCTTT | SEQ ID NO: 179 |
| 52 | ACGGATCTCCAACTAA AATAC | SEQ ID NO: 61 | GTATTTTAGTTGGAGATCC GTTT | SEQ ID NO: 180 |
| 52' | ACGGATCTCCAACTAA AATAT | SEQ ID NO: 62 | ATATTTTAGTTGGAGATCC GTTT | SEQ ID NO: 181 |
| 53 | AACTAAAATACTTCAC GGGAG | SEQ ID NO: 63 | CTCCCGTGAAGTATTTTAG TTGG | SEQ ID NO: 182 |
| 54 | CTTTGTCTCCTTAAAAC ATCT | SEQ ID NO: 64 | AGATGTTTTAAGGAGACA AAGAT | SEQ ID NO: 183 |
| 55 | TTGCCCAGTACACGCA TTAAA | SEQ ID NO: 65 | TTTAATGCGTGTACTGGGC AATC | SEQ ID NO: 184 |
| 56 | CCAGTACACGCATTAA AAAGA | SEQ ID NO: 66 | TCTTTTTAATGCGTGTACT GGGC | SEQ ID NO: 185 |
| 57 | CAGTACACGCATTAAA AAGAG | SEQ ID NO: 67 | CTCTTTTTAATGCGTGTAC TGGG | SEQ ID NO: 186 |
| 58 | TCAGTCTACAAAGCTG CAGGC | SEQ ID NO: 68 | GCCTGCAGCTTTGTAGACT GAAA | SEQ ID NO: 187 |
| 59 | TAAGCCAACAAGGTCT TTTCA | SEQ ID NO: 69 | TGAAAAGACCTTGTTGGC TTACT | SEQ ID NO: 188 |
| 60 | AAGCCAACAAGGTCTT TTCAG | SEQ ID NO: 70 | CTGAAAAGACCTTGTTGG CTTAC | SEQ ID NO: 189 |
| 61 | GAGGGGACATTACTAC GGTCT | SEQ ID NO: 71 | AGACCGTAGTAATGTCCC CTCCT | SEQ ID NO: 190 |
| 62 | GGGGACATTACTACGG TCTTC | SEQ ID NO: 72 | GAAGACCGTAGTAATGTC CCCTC | SEQ ID NO: 191 |
| 63 | GACATTACTACGGTCTT CACA | SEQ ID NO: 73 | TGTGAAGACCGTAGTAAT GTCCC | SEQ ID NO: 192 |
| 64 | TCTTCACTTTCACGGCG GAAT | SEQ ID NO: 74 | ATTCCGCCGTGAAAGTGA AGAGT | SEQ ID NO: 193 |
| 65 | CTTCACTTTCACGGCGG AATC | SEQ ID NO: 75 | GATTCCGCCGTGAAAGTG AAGAG | SEQ ID NO: 194 |
| 66 | TTCACTTTCACGGCGGA ATCA | SEQ ID NO: 76 | TGATTCCGCCGTGAAAGT GAAGA | SEQ ID NO: 195 |
| 67 | GATCCCACCCGATGGT TTACT | SEQ ID NO: 77 | AGTAAACCATCGGGTGGG ATCCT | SEQ ID NO: 196 |
| 68 | CCAAGAGTGAATAGGA CAGCA | SEQ ID NO: 78 | TGCTGTCCTATTCACTCTT GGCA | SEQ ID NO: 197 |
| 69 | ATAGGACAGCAGCGAT TTCTG | SEQ ID NO: 79 | CAGAAATCGCTGCTGTCC TATTC | SEQ ID NO: 198 |
| 69' | ATAGGACAGCAGCGAT TTCTT | SEQ ID NO: 80 | AAGAAATCGCTGCTGTCC TATTC | SEQ ID NO: 199 |
| 70 | CACCAAATAAGCGCTT GCAAC | SEQ ID NO: 81 | GTTGCAAGCGCTTATTTGG TGTG | SEQ ID NO: 200 |
| 70' | CACCAAATAAGCGCTT GCAAT | SEQ ID NO: 82 | ATTGCAAGCGCTTATTTGG TGTG | SEQ ID NO: 201 |
| 71 | AAAGATGCACGGATGA CGTCC | SEQ ID NO: 83 | GGACGTCATCCGTGCATC TTTCT | SEQ ID NO: 202 |
| 72 | GATACACATTAAGGTT GTGTA | SEQ ID NO: 84 | TACACAACCTTAATGTGTA TCCA | SEQ ID NO: 203 |
| 73 | ATTGCCCAGTACACGC ATTAA | SEQ ID NO: 85 | TTAATGCGTGTACTGGGC AATCC | SEQ ID NO: 204 |
| 74 | AAAGCTCTTTCTGGTTT CAGT | SEQ ID NO: 86 | ACTGAAACCAGAAAGAGC TTTGC | SEQ ID NO: 205 |
| 75 | TGAAGGAGGGGACATT ACTAC | SEQ ID NO: 87 | GTAGTAATGTCCCCTCCTT CAAA | SEQ ID NO: 206 |
| 76 | CATTACTACGGTCTTCA CACC | SEQ ID NO: 88 | GGTGTGAAGACCGTAGTA ATGTC | SEQ ID NO: 207 |
| 77 | CACACCAAGCGCCAAG TACTG | SEQ ID NO: 89 | CAGTACTTGGCGCTTGGT GTGAA | SEQ ID NO: 208 |
| 78 | CTCTTCACTTTCACGGC GGAA | SEQ ID NO: 90 | TTCCGCCGTGAAAGTGAA GAGTA | SEQ ID NO: 209 |
| 79 | TCACTTTCACGGCGGA ATCAC | SEQ ID NO: 91 | GTGATTCCGCCGTGAAAG TGAAG | SEQ ID NO: 210 |
| 80 | TCCCACCCGATGGTTTA CTTG | SEQ ID NO: 92 | CAAGTAAACCATCGGGTG GGATC | SEQ ID NO: 211 |
| 81 | AGAGTGAATAGGACAG CAGCG | SEQ ID NO: 93 | CGCTGCTGTCCTATTCACT CTTG | SEQ ID NO: 212 |
| 82 | AGTGAATAGGACAGCA GCGAT | SEQ ID NO: 94 | ATCGCTGCTGTCCTATTCA CTCT | SEQ ID NO: 213 |
| 84 | AATAGGACAGCAGCGA TTTCT | SEQ ID NO: 95 | AGAAATCGCTGCTGTCCT ATTCA | SEQ ID NO: 214 |
| 83 | GCATAAACTATAACAG CTCAG | SEQ ID NO: 96 | CTGAGCTGTTATAGTTTAT GCCC | SEQ ID NO: 215 |
| 85 | GGAAGAAAAGCACCCT TATTA | SEQ ID NO: 97 | TAATAAGGGTGCTTTTCTT CCTC | SEQ ID NO: 216 |
| 86 | GAAGAAAAGCACCCTT ATTAA | SEQ ID NO: 98 | TTAATAAGGGTGCTTTTCT TCCT | SEQ ID NO: 217 |
| 87 | TTTTTCACGTACGCCAC AAGG | SEQ ID NO: 99 | CCTTGTGGCGTACGTGAA AAAGT | SEQ ID NO: 218 |
| 88 | TTTTCACGTACGCCACA AGGC | SEQ ID NO: 100 | GCCTTGTGGCGTACGTGA AAAAG | SEQ ID NO: 219 |
| 89 | AGAAAAGCACCCTTAT TAAGA | SEQ ID NO: 101 | TCTTAATAAGGGTGCTTTT CTTC | SEQ ID NO: 220 |
| 90 | ATTGCAGCAAGTAAGC CAACA | SEQ ID NO: 102 | TGTTGGCTTACTTGCTGCA ATTC | SEQ ID NO: 221 |
| 91 | TCTCCTTAAAACATCTG AGAG | SEQ ID NO: 103 | CTCTCAGATGTTTTAAGGA GACA | SEQ ID NO: 222 |
| 92 | GACTCAGTTGTTGAAG GACAC | SEQ ID NO: 104 | GTGTCCTTCAACAACTGA GTCAC | SEQ ID NO: 223 |
| 93 | TTGAAGGAGGGGACAT TACTA | SEQ ID NO: 105 | TAGTAATGTCCCCTCCTTC AAAG | SEQ ID NO: 224 |
| 94 | ACTCTTCACTTTCACGG CGGA | SEQ ID NO: 106 | TCCGCCGTGAAAGTGAAG AGTAA | SEQ ID NO: 225 |
| 95 | GGACAGCAGCGATTTC TGGGT | SEQ ID NO: 107 | ACCCAGAAATCGCTGCTG TCCTA | SEQ ID NO: 226 |
| 96 | CATAAACTATAACAGC TCAGT | SEQ ID NO: 108 | ACTGAGCTGTTATAGTTTA TGCC | SEQ ID NO: 227 |
| 97 | AACTATAACAGCTCAG TTGCC | SEQ ID NO: 109 | GGCAACTGAGCTGTTATA GTTTA | SEQ ID NO: 228 |
| 98 | AAGAAAAGCACCCTTA TTAAG | SEQ ID NO: 110 | CTTAATAAGGGTGCTTTTC TTCC | SEQ ID NO: 229 |
| 99 | AGCAAGTAAGCCAACA AGGTC | SEQ ID NO: 111 | GACCTTGTTGGCTTACTTG CTGC | SEQ ID NO: 230 |
| 100 | GCAAGTAAGCCAACAA GGTCT | SEQ ID NO: 112 | AGACCTTGTTGGCTTACTT GCTG | SEQ ID NO: 231 |
| RB2 | GGGTATTCTTTCAAGCA AT | SEQ ID NO: 113 | ATTGCTTGAAAGAATACC CAG | SEQ ID NO: 232 |
| RB4 | GCTCAAGAATGCCAAG AAA | SEQ ID NO: 114 | TTTCTTGGCATTCTTGAGC AC | SEQ ID NO: 233 |
| RB6 | GAATCTCAAAGAAATC TTT | SEQ ID NO: 115 | AAAGATTTCTTTGAGATTC TT | SEQ ID NO: 234 |
| RB7 | ATTTCTGGGTATTCTTT CA | SEQ ID NO: 116 | TGAAAGAATACCCAGAAA TCG | SEQ ID NO: 235 |
| TJ632 | AAGCAATGCTCACACC AAA | SEQ ID NO: 117 | TTTGGTGTGAGCATTGCTT GA | SEQ ID NO: 236 |
| TJ645 | CTTGCAACAAAGACAT TTA | SEQ ID NO: 118 | TAAATGTCTTTGTTGCAAG CG | SEQ ID NO: 237 |
| TJ772 | GGATATTGTTGCTGCAA AA | SEQ ID NO: 119 | TTTTGCAGCAACAATATCC AG | SEQ ID NO: 238 |
| ISIS-416858 | - | - | ACGGCATTGGTGCACAGT TT | SEQ ID NO: 239 |

| qPCR primers | | | | |
|---|---|---|---|---|
| Primer name | Upstream primer sequence | Upstrea m primer sequenc e ID | Downstream primer sequence | Downstrea m primer sequence ID |
| Human - GAPD H | GATTTGGTCGTATTGGG CGC | SEQ ID NO: 240 | TTCCCGTTCTCAGCCTTGA C | SEQ ID NO: 241 |
| Human -FXI | TCACTTTCACGGCGGA ATCA | SEQ ID NO: 242 | TCCGTGCATCTTTCTTGGC A | SEQ ID NO: 243 |

| Reference sequence: FXI mRNA sequence | | | | |
|---|---|---|---|---|
| NM_00 0128.4 | gaccatttcctacacaagctcattcagaggaggatgaagaccattttggaggaagaaa agcacccttattaagaattgcagcaagtaagccaacaaggtcttttcaggatgattttctt atatcaagtggtacatttcattttatttacttcagtttctggtgaatgtgtgactcagttgttg aaggacacctgctttgaaggaggggacattactacggtcttcacaccaagcgccaagt actgccaggtagtctgcacttaccacccaagatgtttactcttcactttcacggcggaatc accatctgaggatcccacccgatggtttacttgtgtcctgaaagacagtgttacagaaa cactgccaagagtgaataggacagcagcgatttctgggtattctttcaagcaatgctca | | | SEQ ID NO: 244 |
| | caccaaataagcgcttgcaacaaagacatttatgtggacctagacatgaagggcataa actataacagctcagttgccaagagtgctcaagaatgccaagaaagatgcacggatg acgtccactgccactttttcacgtacgccacaaggcagtttcccagcctggagcatcgta acatttgtctactgaagcacacccaaacagggacaccaaccagaataacgaagctcg ataaagtggtgtctggattttcactgaaatcctgtgcactttctaatctggcttgtattagg gacattttccctaatacggtgtttgcagacagcaacatcgacagtgtcatggctcccgat gcttttgtctgtggccgaatctgcactcatcatcccggttgcttgtttttttaccttcttttccca ggaatggcccaaagaatctcaaagaaatctttgtctccttaaaacatctgagagtggat tgcccagtacacgcattaaaaagagcaaagctctttctggtttcagtctacaaagctgca ggcacagcatcccagtgttctgccattcttcattttaccatgacactgatttcttgggaga agaactggatattgttgctgcaaaaagtcacgaggcctgccagaaactgtgcaccaat gccgtccgctgccagtttttttacctataccccagcccaagcatcctgcaacgaagggaa gggcaagtgttacttaaagctttcttcaaacggatctccaactaaaatacttcacgggag aggaggcatctctggatacacattaaggttgtgtaaaatggataatgagtgtaccacc aaaatcaagcccaggatcgttggaggaactgcgtctgttcgtggtgagtggccgtgg caggtgaccctgcacacaacctcacccactcagagacacctgtgtggaggctccatca ttggaaaccagtggatattaacagccgctcactgtttctatggggtagagtcacctaag attttgcgtgtctacagtggcattttaaatcaatctgaaataaaagaggacacatctttctt tggggttcaagaaataataatccatgatcagtataaaatggcagaaagcgggtatgat attgccttgttgaaactggaaaccacagtgaattacacagattctcaacgacccatatgc ctgccttccaaaggagatagaaatgtaatatacactgattgctgggtgactggatggg ggtacagaaaactaagagacaaaatacaaaatactctccagaaagccaagatacccctt agtgaccaacgaagagtgccagaagagatacagaggacataaaataacccataaga tgatctgtgccggctacagggaaggagggaaggacgcttgcaagggagattcggg aggccctctgtcctgcaaacacaatgaggtctggcatctggtaggcatcacgagctgg ggcgaaggctgtgctcaaagggagcggccaggtgtttacaccaacgtggtcgagta cgtggactggattctggagaaaactcaagcagtgtgaatgggttcccaggggccatt ggagtccctgaaggacccaggatttgctgggagagggtgttgagttcactgtgccag catgcttcctccacagtaacacgctgaaggggcttggtgtttgtaagaaaatgctagaa gaaaacaaactgtcacaagttgttatgtccaaaactcccgttctatgatcgttgtagtttgt ttgagcattcagtctctttgtttttgatcacgcttctatggagtccaagaattaccataagg caatatttctgaagattactatataggcagatatagcagaaaataaccaagtagtggca gtggggatcaggcagaagaactggtaaaagaagccaccataaatagatttgttcgat gaaagatgaaaactggaagaaaggagaacaaagacagtcttcaccattttgcagga atctacactctgcctatgtgaacacatttcttttgtaaagaaagaaattgattgcatttaat ggcagattttcagaatagtcaggaattcttgtcatttccattttaaaatatatattaaaaaa aatcagttcgagtagacacgagctaagagtgaatgtgaagataacagaatttctgtgt ggaagaggattacaagcagcaatttacctggaagtgataccttaggggcaatcttgaa gatacactttcctgaaaaatgatttgtgatggattgtatatttatttaaaatatcttgggag gggaggctgatggagatagggagcatgctcaaacctccctaagacaagctgctgct gtgactatgggctcccaaagagctagatcgtatatttatttgacaaaaatcaccatagac tgcatccatactacagagaaaaaacaattagggcgcaaatggatagttacagtaaagt cttcagcaagcagctgcctgtattctaagcactgggattttctgtttcgtgcaaatatttat ctcattattgttgtgatctagttcaataacctagaatttgaattgtcaccacatagctttcaa tctgtgccaacaactatacaattcatcaagtgtg | | | |

## Claims

1. A nucleic acid molecule for inhibiting the expression of factor 11 (FXI) gene in cells via RNAi, comprising or consisting of a sense sequence and an antisense sequence complementary to each other, with the antisense sequence comprising a polynucleotide sequence complementary to FXI gene mRNA, wherein
when the antisense sequence hybridizes with the FXI gene mRNA at the maximum complementarity, the base pairs formed by complementary pairing between the antisense sequence and the FXI gene mRNA sequence constitute complementary region 1;
when the antisense sequence hybridizes with the sense sequence at the maximum complementarity, the base pairs formed by complementary pairing between the antisense sequence and the sense sequence constitute complementary region 2;
the complementary region 1 and the complementary region 2 share at least 18, 19 or 20 identical base pairs,
the number of base pairs in the complementary region 1 is 18 to 30 bp, and the complementary region 1 comprises the following nucleotides in the FXI gene mRNA sequence:
nucleotides at positions 418 to 437, or 18 or 19 consecutive nucleotides therein,
nucleotides at positions 1141 to 1160, or 18 or 19 consecutive nucleotides therein,
nucleotides at positions 363 to 382, or 18 or 19 consecutive nucleotides therein,
nucleotides at positions 372 to 391, or 18 or 19 consecutive nucleotides therein,
nucleotides at positions 411 to 430, or 18 or 19 consecutive nucleotides therein,
nucleotides at positions 421 to 440, or 18 or 19 consecutive nucleotides therein,
nucleotides at positions 422 to 441, or 18 or 19 consecutive nucleotides therein,
nucleotides at positions 425 to 444, or 18 or 19 consecutive nucleotides therein,
nucleotides at positions 465 to 484, or 18 or 19 consecutive nucleotides therein,
nucleotides at positions 470 to 489, or 18 or 19 consecutive nucleotides therein,
nucleotides at positions 471 to 490, or 18 or 19 consecutive nucleotides therein, or
nucleotides at positions 518 to 537, or 18 or 19 consecutive nucleotides therein; and
the nucleotide positions of the FXI gene mRNA sequence are numbered in accordance with the corresponding nucleotides in the reference sequence of SEQ ID NO: 244.

2. The nucleic acid molecule according to claim 1, wherein the number of base pairs in the complementary region 1 is 20, 21, 22 or 23 bp.

3. The nucleic acid molecule according to claim 1 or 2, wherein the number of base pairs in the complementary region 2 is 21 bp.

4. The nucleic acid molecule according to any one of claims 1 to 3, wherein the complementary region 1 and the complementary region 2 have 20 or 21 identical base pairs.

5. The nucleic acid molecule according to any one of claims 1 to 4, wherein the length of the sense sequence is 21 nt and the length of the antisense sequence is 23 nt.

6. The nucleic acid molecule according to any one of claims 1 to 5, wherein the sense sequence and/or antisense sequence further comprises 1 to 2 overhang nucleotides outside the complementary region 2.

7. The nucleic acid molecule according to claim 6, wherein there are 2 overhang nucleotides located in the antisense sequence and adjacent to the 5' end of the complementary region 2, and the sense sequence comprises no overhang nucleotides.

8. The nucleic acid molecule according to any one of claims 1 to 7, wherein the antisense sequence comprises only 0, 1 or 2 nucleotides outside the complementary region 1, and the sense sequence comprises only 0, 1 or 2 nucleotides outside the complementary region 2.

9. The nucleic acid molecule according to any one of claims 1 to 8, wherein the 3' end of the complementary region 2 is an AU base pair.

10. The nucleic acid molecule according to any one of claims 1 to 9, wherein the FXI gene is a human FXI gene or a cynomolgus monkey FXI gene.

11. The nucleic acid molecule according to claim 10, wherein the mRNA comprises the polynucleotide sequence set forth in SEQ ID NO: 244.

12. The nucleic acid molecule according to any one of claims 1 to 11, wherein the antisense sequence comprises or is the polynucleotide sequence set forth in SEQ ID NO: 153, 177, 152, 122, 123, 126, 129, 130, 131, 136, 137, 138, 143, 149, 150, 200, 201 or 213.

13. The nucleic acid molecule according to any one of claims 1 to 12, wherein:
the sense sequence comprises or is the polynucleotide sequence set forth in SEQ ID NO: 34, and the antisense sequence comprises or is the polynucleotide sequence set forth in SEQ ID NO: 153;
the sense sequence comprises or is the polynucleotide sequence set forth in SEQ ID NO: 58, and the antisense sequence comprises or is the polynucleotide sequence set forth in SEQ ID NO: 177; the sense sequence comprises or is the polynucleotide sequence set forth in SEQ ID NO: 33, and the antisense sequence comprises or is the polynucleotide sequence set forth in SEQ ID NO: 152;
the sense sequence comprises or is the polynucleotide sequence set forth in SEQ ID NO: 3, and the antisense sequence comprises or is the polynucleotide sequence set forth in SEQ ID NO: 122;
the sense sequence comprises or is the polynucleotide sequence set forth in SEQ ID NO: 4, and the antisense sequence comprises or is the polynucleotide sequence set forth in SEQ ID NO: 123;
the sense sequence comprises or is the polynucleotide sequence set forth in SEQ ID NO: 7, and the antisense sequence comprises or is the polynucleotide sequence set forth in SEQ ID NO: 126;
the sense sequence comprises or is the polynucleotide sequence set forth in SEQ ID NO: 10, and the antisense sequence comprises or is the polynucleotide sequence set forth in SEQ ID NO: 129;
the sense sequence comprises or is the polynucleotide sequence set forth in SEQ ID NO: 11, and the antisense sequence comprises or is the polynucleotide sequence set forth in SEQ ID NO: 130;
the sense sequence comprises or is the polynucleotide sequence set forth in SEQ ID NO: 12, and the antisense sequence comprises or is the polynucleotide sequence set forth in SEQ ID NO: 131;
the sense sequence comprises or is the polynucleotide sequence set forth in SEQ ID NO: 17, and the antisense sequence comprises or is the polynucleotide sequence set forth in SEQ ID NO: 136;
the sense sequence comprises or is the polynucleotide sequence set forth in SEQ ID NO: 18, and the antisense sequence comprises or is the polynucleotide sequence set forth in SEQ ID NO: 137;
the sense sequence comprises or is the polynucleotide sequence set forth in SEQ ID NO: 19, and the antisense sequence comprises or is the polynucleotide sequence set forth in SEQ ID NO: 138;
the sense sequence comprises or is the polynucleotide sequence set forth in SEQ ID NO: 24, and the antisense sequence comprises or is the polynucleotide sequence set forth in SEQ ID NO: 143;
the sense sequence comprises or is the polynucleotide sequence set forth in SEQ ID NO: 30, and the antisense sequence comprises or is the polynucleotide sequence set forth in SEQ ID NO: 149;
the sense sequence comprises or is the polynucleotide sequence set forth in SEQ ID NO: 31, and the antisense sequence comprises or is the polynucleotide sequence set forth in SEQ ID NO: 150;
the sense sequence comprises or is the polynucleotide sequence set forth in SEQ ID NO: 81, and the antisense sequence comprises or is the polynucleotide sequence set forth in SEQ ID NO: 200;
the sense sequence comprises or is the polynucleotide sequence set forth in SEQ ID NO: 82, and the antisense sequence comprises or is the polynucleotide sequence set forth in SEQ ID NO: 201; or
the sense sequence comprises or is the polynucleotide sequence set forth in SEQ ID NO: 94, and the antisense sequence comprises or is the polynucleotide sequence set forth in SEQ ID NO: 213.

14. The nucleic acid molecule according to any one of claims 1 to 13, wherein the nucleic acid molecule is a dsRNA or shRNA.

15. The nucleic acid molecule according to claim 14, wherein the nucleic acid molecule is an siRNA.

16. The nucleic acid molecule according to any one of claims 1 to 15, wherein one or more nucleotides are chemically modified.

17. The nucleic acid molecule according to any one of claims 1 to 16, wherein the chemical modification is one or more selected from the group consisting of:
locked nucleic acid modification, unlocked nucleic acid modification, 2'-methoxyethyl modification, 2'-O-methyl modification, 2'-O-allyl modification, 2'-C-alkyl modification, 2'-C-allyl modification, 2'-fluoro modification, 2'-deoxy modification, phosphorothioate bond modification, 2'-amino modification, morpholino modification, phosphoramidate modification, methylphosphonate modification, tetrahydropyran modification, 1,5-anhydrohexitol modification, 5'-vinyl phosphate modification, and cyclohexenyl modification;
preferably, in the nucleic acid molecule, the nucleotides at positions 7, 9 to 12 of the sense strand comprise 2'-fluoro modifications and/or the nucleotides at positions 2, 14 and 16 of the antisense strand comprise 2'-fluoro modifications, and further preferably, the remaining nucleotides comprise 2'-O-methyl modifications;
preferably, in the nucleic acid molecule, the nucleotides at positions 7, 9 to 11 of the sense strand comprise 2'-fluoro modifications and/or the nucleotides at positions 2, 14 and 16 of the antisense strand comprise 2'-fluoro modifications; and further preferably, the remaining nucleotides comprise 2'-O-methyl modifications;
preferably, in the nucleic acid molecule, the nucleotides at positions 7, 9 to 11 of the sense strand comprise 2'-fluoro modifications, and the nucleotides at positions 2, 6, 14 and 16 of the antisense strand comprise 2'-fluoro modifications; and further preferably, the remaining nucleotides comprise 2'-O-methyl modifications;
preferably, the nucleic acid molecule comprises a motif selected from the group consisting of:
(1) sense sequence: NmNmNmNmNmNmNfNmNfNfNfNfNmNmNmNmNmNmNmNmNm, antisense sequence: NmNfNmNmNmNmNmNmNmNmNmNmNmNfNmNfNmNmNmNmNmNmNm,
(2) sense sequence: NmNmNmNmNmNmNfNmNfNfNfNmNmNmNmNmNmNmNmNmNm, antisense sequence: NmNfNmNmNmNmNmNmNmNmNmNmNmNfNmNfNmNmNmNmNmNmNm, and
(3) sense sequence: NmNmNmNmNmNmNfNmNfNfNfNmNmNmNmNmNmNmNmNmNm, antisense sequence: NmNfNmNmNmNfNmNmNmNmNmNmNmNfNmNfNmNmNmNmNmNmNm,
wherein Nm represents a ribonucleotide modified with 2'-O-methyl, and Nf represents a ribonucleotide modified with 2'-fluoro;
further preferably, numbered from the 5' end of the sense strand, at least one of the following positions has a phosphorothioate modification: between the first and second nucleotides, between the second and third nucleotides, between the last and penultimate nucleotides, and between the penultimate and antepenultimate nucleotides; numbered from the 5' end of the antisense strand, at least one of the following positions has a phosphorothioate modification: between the first and second nucleotides, between the second and third nucleotides, between the last and penultimate nucleotides, and between the penultimate and antepenultimate nucleotides.

18. The nucleic acid molecule according to claim 17, wherein the nucleic acid molecule comprises a motif selected from the following group consisting of:
(1) sense sequence: NmsNmsNmNmNmNmNfNmNfNfNfNfNmNmNmNmNmNmNmNmNm, antisense sequence: NmsNfsNmNmNmNmNmNmNmNmNmNmNmNfNmNfNmNmNmNmNmsNmsNm;
(2) sense sequence: NmsNmsNmNmNmNmNfNmNfNfNfNmNmNmNmNmNmNmNmNmNm, antisense sequence: NmsNfsNmNmNmNmNmNmNmNmNmNmNmNfNmNfNmNmNmNmNmsNmsNm;
(3) sense sequence: NmsNmsNmNmNmNmNfNmNfNfNfNmNmNmNmNmNmNmNmNmNm, antisense sequence: NmsNfsNmNmNmNfNmNmNmNmNmNmNmNfNmNfNmNmNmNmNmsNmsNm;
(4) sense sequence: NmsNmsNmNmNmNmNfNmNfNfNfNfNmNmNmNmNmNmNmsNmsNm, antisense sequence: NmsNfsNmNmNmNmNmNmNmNmNmNmNmNfNmNfNmNmNmNmNmsNmsNm;
(5) sense sequence: NmsNmsNmNmNmNmNfNmNfNfNfNmNmNmNmNmNmNmNmsNmsNm, antisense sequence: NmsNfsNmNmNmNmNmNmNmNmNmNmNmNfNmNfNmNmNmNmNmsNmsNm; and
(6) sense sequence: NmsNmsNmNmNmNmNfNmNfNfNfNmNmNmNmNmNmNmNmsNmsNm, antisense sequence: NmsNfsNmNmNmNfNmNmNmNmNmNmNmNfNmNfNmNmNmNmNmsNmsNm;
(7) sense sequence: NmsNmsNmNmNmNmNfNmNfNfNfNfNmNmNmNmNmNmNmNmNms, antisense sequence: NmsNfsNmNmNmNmNmNmNmNmNmNmNmNfNmNfNmNmNmNmNmsNmsNm;
(8) sense sequence: NmsNmsNmNmNmNmNfNmNfNfNfNmNmNmNmNmNmNmNmNmNms, antisense sequence: NmsNfsNmNmNmNmNmNmNmNmNmNmNmNfNmNfNmNmNmNmNmsNmsNm;
(9) sense sequence: NmsNmsNmNmNmNmNfNmNfNfNfNmNmNmNmNmNmNmNmNmNms, antisense sequence: NmsNfsNmNmNmNfNmNmNmNmNmNmNmNfNmNfNmNmNmNmNmsNmsNm;
wherein Nm represents a ribonucleotide modified with 2'-O-methyl, Nf represents a ribonucleotide modified with 2'-fluoro, and s represents a phosphorothioate modification.

19. The nucleic acid molecule according to any one of claims 1 to 18, wherein the nucleic acid molecule is linked to at least one asialoglycoprotein receptor (ASGPR) ligand.

20. The nucleic acid molecule according to claim 19, wherein the ASGPR ligand is linked to the 5' end or 3' end of the sense sequence.

21. The nucleic acid molecule according to claim 19 or 20, wherein the ASGPR ligand is one or more GalNAc derivatives linked via a divalent or trivalent branched structure.

22. The nucleic acid molecule according to claim 21, wherein the GalNAc derivative is L96 or ligand 1, and the structure of the L96 is shown below: 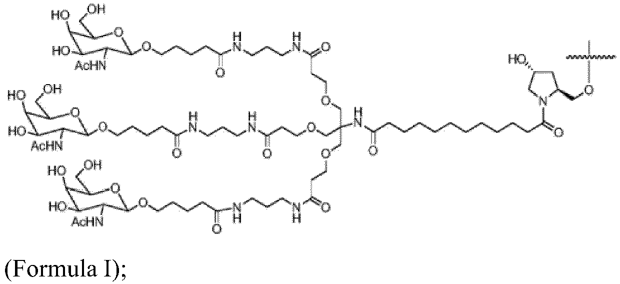
the structure of the ligand 1 is shown below: 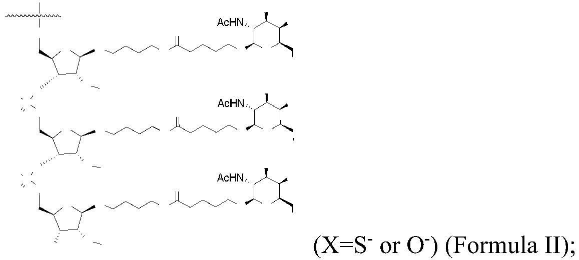
wherein in each of formula I and formula II represents linkage to the 3' end of the sense sequence or antisense sequence of the nucleic acid molecule; preferably, the linkage is formed via a phosphodiester bond or a phosphorothioate diester bond to 3'-hydroxy;
further preferably, the nucleic acid molecule has any one of the following modification motifs:
(1) sense sequence: NmsNmsNmNmNmNmNfNmNfNfNfNfNmNmNmNmNmNmNmNmNm-L96; antisense sequence: NmsNfsNmNmNmNmNmNmNmNmNmNmNmNfNmNfNmNmNmNmNmsNmsNm;
(2) sense sequence: NmsNmsNmNmNmNmNfNmNfNfNfNfNmNmNmNmNmNmNmNmNms-ligand 1; antisense sequence: NmsNfsNmNmNmNmNmNmNmNmNmNmNmNfNmNfNmNmNmNmNmsNmsNm;
(3) sense sequence: NmsNmsNmNmNmNmNfNmNfNfNfNmNmNmNmNmNmNmNmNmNm-L96; antisense sequence: NmsNfsNmNmNmNfNmNmNmNmNmNmNmNfNmNfNmNmNmNmNmsNmsNm;
(4) sense sequence: NmsNmsNmNmNmNmNfNmNfNfNfNmNmNmNmNmNmNmNmNmNms-ligand 1; antisense sequence: NmsNfsNmNmNmNfNmNmNmNmNmNmNmNfNmNfNmNmNmNmNmsNmsNm;
(5) sense sequence: NmsNmsNmNmNmNmNfNmNfNfNfNmNmNmNmNmNmNmNmNmNm-L96; antisense sequence: NmsNfsNmNmNmNmNmNmNmNmNmNmNmNfNmNfNmNmNmNmNmsNmsNm;
(6) sense sequence: NmsNmsNmNmNmNmNfNmNfNfNfNmNmNmNmNmNmNmNmNmNms-ligand 1; antisense sequence: NmsNfsNmNmNmNmNmNmNmNmNmNmNmNfNmNfNmNmNmNmNmsNmsNm.

23. The nucleic acid molecule according to any one of claims 1 to 22, wherein the nucleic acid molecule comprises a phosphate or phosphate mimetic modification at the 5' end of the antisense strand; preferably, the 5' phosphate mimetic modification is 5'-VP;
further preferably, the nucleic acid molecule has any one of the following modification motifs:
GN-E20VP:
sense sequence: NmsNmsNmNmNmNmNfNmNfNfNfNfNmNmNmNmNmNmNmNmNms-ligand 1;
antisense sequence: vp-NmsNfsNmNmNmNmNmNmNmNmNmNmNmNfNmNfNmNmNmNmNmsNmsNm;
GN-E04VP:
sense sequence: NmsNmsNmNmNmNmNfNmNfNfNfNmNmNmNmNmNmNmNmNmNms-ligand 1;
antisense sequence: vp-NmsNfsNmNmNmNfNmNmNmNmNmNmNmNfNmNfNmNmNmNmNmsNmsNm;
GN-E05VP:
sense sequence: NmsNmsNmNmNmNmNfNmNfNfNfNmNmNmNmNmNmNmNmNmNms-ligand 1;
antisense sequence: vp-NmsNfsNmNmNmNmNmNmNmNmNmNmNmNfNmNfNmNmNmNmNmsNmsNm.

24. A second nucleic acid molecule, wherein the second nucleic acid molecule can be transcribed into a dsRNA or shRNA precursor in a cell, and the dsRNA or shRNA is the nucleic acid molecule according to any one of claims 1 to 23.

25. A viral particle, comprising the polynucleotide sequence of the second nucleic acid molecule according to claim 24.

26. A cell, comprising the polynucleotide sequence of the second nucleic acid molecule according to claim 24.

27. Use of the nucleic acid molecule according to any one of claims 1 to 23 or a salt thereof for inhibiting the expression of factor 11.

28. Use of the nucleic acid molecule according to any one of claims 1 to 23 or a salt thereof in the manufacture of a drug for inhibiting the expression of FXI in a subject.

29. A pharmaceutical composition, comprising the nucleic acid molecule according to any one of claims 1 to 23 or a salt thereof, the second nucleic acid molecule according to claim 24, the viral particle according to claim 25, or the cell according to claim 26.

30. Use of the nucleic acid molecule according to any one of claims 1 to 23 or a salt thereof, the second nucleic acid molecule according to claim 24, the viral particle according to claim 25, the cell according to claim 26, or the pharmaceutical composition according to claim 29 in the manufacture of a drug for preventing or treating thromboembolic complications or coagulation disorders in a subject; preferably, the thromboembolic complications are one or more selected from the group consisting of deep vein thrombosis, pulmonary embolism, myocardial infarction and stroke.

31. A method for preventing or treating thromboembolic complications or coagulation disorders in a subject, wherein the thromboembolic complications are preferably one or more selected from the group consisting of deep vein thrombosis, pulmonary embolism, myocardial infarction and stroke, and the method comprises administering an effective amount of the nucleic acid molecule according to any one of claims 1 to 23 or a salt thereof, the second nucleic acid molecule according to claim 24, the viral particle according to claim 25, the cell according to claim 26, or the pharmaceutical composition according to claim 29 to a subject in need thereof.
